Europäisches Patentamt

**European Patent Office**

**Office européen des brevets**

(11) Numéro de publication: **0 298 864 B1**

(12) **FASCICULE DE BREVET EUROPEEN**

(45) Date de publication de fascicule du brevet: **17.06.92**

(51) Int. Cl.5: **B01D 39/16**, B01D 46/00, B01J 13/02, B01J 2/04, B01D 1/18

(21) Numéro de dépôt: **88401764.1**

(22) Date de dépôt: **06.07.88**

(54) **Filtre comprenant un matériau obtenu par cryodessiccation, procédé de préparation et utilisation notamment en galénique.**

(30) Priorité: **08.07.87 FR 8709696**
**12.08.87 FR 8711468**
**04.09.87 FR 8712277**

(43) Date de publication de la demande:
**11.01.89 Bulletin 89/02**

(45) Mention de la délivrance du brevet:
**17.06.92 Bulletin 92/25**

(84) Etats contractants désignés:
**AT BE CH DE ES FR GB GR IT LI LU NL SE**

(56) Documents cités:
**EP-A- 0 227 486       WO-A-85/05288**
**FR-A- 1 566 272       US-A- 3 190 765**
**US-A- 4 155 247       US-A- 4 351 849**

**EXPERIENTIA, vol. 21, no. 5, 15 mai 1965, pages 241-246; L. REY: "Un développement nouveau de la lyophilisation: la cryodessic-cation des systèmes non aqueux"**

(73) Titulaire: **LABORATOIRE L. LAFON**
**19 Avenue du Professeur Cadiot**
**F-94701 Maisons Alfort(FR)**

(72) Inventeur: **Rey, Louis**
**La Jacquière**
**CH-1066 Epalinges(CH)**
Inventeur: **Ottar, Brynjulf**
**Soerum - Terrace 43**
**N-2007 Kjeller(NO)**
Inventeur: **Lafon, Louis**
**5, rue de l'Alboni**
**F-75016 Paris(FR)**

(74) Mandataire: **Clisci, Serge et al**
**S.A. FEDIT-LORIOT CONSEILS EN PROPRIETE INDUSTRIELLE 38, avenue Hoche**
**F-75008 Paris(FR)**

Rank Xerox (UK) Business Services

## Description

## DOMAINE DE L'INVENTION

La présente invention a trait au domaine de la filtration. Elle concerne plus particulièrement (i) un filtre comportant un élément ou moyen filtrant obtenu par cryodessiccation ou lyophilisation puis compression, (ii) le procédé de préparation de ce filtre et dudit élément filtrant, et (iii) l'utilisation dudit filtre et dudit élément filtrant dans le domaine de la rétention sélective de produits gazeux ou particulaires à des fins séparatives, analytiques et/ou préparatives, d'une part, et dans le domaine de l'obtention de formes galéniques comprenant ledit élément filtrant en tant que matrice en association avec des microparticules de principes actifs, d'autre part.

## ART ANTERIEUR

On sait que dans le domaine de la filtration on a déjà utilisé dans le passé des filtres en cellulose, polyuréthane, métal fritté, verre fritté, carbone etc. Or il se trouve que ces filtres présentent l'inconvénient de ne pas permettre une récupération complète des produits retenus, en raison notamment des capacités d'adsorption, de rétention et de relargage ou désorption desdits filtres. En particulier, si l'absorption d'un produit sur un filtre implique des forces de liaison intenses, le lavage de ce filtre peut être incomplet quand on veut récupérer ledit produit qui a été retenu. Un tel cas se présente quand on suit l'enseignement de EP-A-0 064 967 selon lequel on prépare des particules submicroniques d'un diamètre inférieur à 600 nm par polymérisation de cyanoacrylate d'alkyle en polycyanoacrylate d'alkyle dans un milieu aqueux contenant une substance biologiquement active puis recueille par filtration sur verre fritté les particules insolubles de polyacrylate d'alkyle revêtues de ladite substance biologiquement active.

Parmi les solutions connues dans le domaine de la filtration de fluides liquides ou gazeux pour retenir de très fines fibres d'amiante ou des particules submicroniques, on sait que l'on a déjà fait appel à des membranes filtrantes de structure microporeuse, notamment des membranes dites "MILLIPORE"[R] commercialisées par la société dite MILLIPORE S.A. de Saint-Quentin-en-Yvelines et des membranes analogues qui proviennent d'autres fabricants. Ces membranes filtrantes microporeuses sont généralement des substances organiques polymères et notamment des ester de cellulose (acétate de cellulose notamment), polyacrylate ou matériau polyacrylique, poly(fluorure de vinylidène), poly(tétrafluorure d'éthylène), polyéthylène, polypropylène ou leurs mélanges. Par ailleurs, pour l'analyse du matériau retenu, lesdites membranes sont généralement carbonisées, et par suite, leur utilisation est généralement limitée à la filtration de fibres et particules minérales non affectées par la carbonisation.

On connait par ailleurs de FR-A-1 566 272 une membrane sèche pour la séparation par diffusion d'un gaz particulier (par exemple l'hélium) d'un mélange gazeux contenant ce gaz particulier (par exemple du gaz dit naturel renfermant environ 2 % molaires d'hélium, 25 % molaires d'azote, 66 % molaires de méthane, le reste étant composé d'éthane de propane et de divers hydrocarbures supérieurs), cette membrane qui est insoluble dans l'eau étant constituée de deux couches : une première couche poreuse en acétate de cellulose d'épaisseur $t_1$ (de l'ordre de 10 à 100$\mu$m) et une seconde couche non poreuse en acétate de cellulose d'épaisseur $t_2$ plus faible (de l'ordre de 0,1 à 1$\mu$m) cette membrane étant obtenue par dépôt à la racle sur un support temporaire, séchage puis lyophilisation de la pellicule résultante. Le brevet FR-A-1 566 272 n'enseigne pas l'obtention d'un matériau poreux obtenu par lyophilisation puis compactage selon l'invention et qui soit utile en tant qu'élément de filtration.

Il existe un besoin en matériau filtrant qui soit soluble dans l'eau ou un autre solvant organique ou minéral usuel et convienne pour retenir les particules submicroniques. En effet, si les filtres spéciaux de laboratoire solubles dans l'eau et notamment conçus en matériau cellulosique non-tissé sont relativement satisfaisants du point de vue analytique dans le domaine de la filtration de produits macroscopiques, ils ne conviennent pas pour la séparation des particules de faibles dimensions, telles que les particules submicroniques, véhiculées par des fluides gazeux.

Il existe par ailleurs un besoin en particules de taille micronique et mieux de taille submicronique pour une utilisation thérapeutique ou cosmétique.

En particulier, on pense sérieusement qu' avec des particules submicroniques, la dégradation enzymatique pourrait, en particulier, être évitée et que certains composés ne possédant pas de transporteurs ou véhicules au niveau des membranes cellulaires pourraient néanmoins être administrés à l'organisme par incorporation selon des processus purement physiques assimilables à la pynocytose.

La fabrication pilote et a fortiori industrielle de particules submicroniques est une opération délicate si l'on tient compte du fait qu'en suspension gazeuse elles ne suivent que les mouvements d'agitation moléculaire ou browniens et sont, de ce fait, très difficiles à recueillir et à concentrer. En outre, il convient d'ajouter qu'en aérosol, la plupart d'entre elles présentent une très grande agressivité à l'égard de l'organisme, leur entrée dans le tractus respiratoire pouvant entraîner dans certains cas des réactions physiologiques indésirables et même présenter une toxicité très élevée, d'autant plus aigüe que la taille particulaire est plus petite.

On recherche donc l'obtention de particules de taille submicronique suivant une technologie appropriée, parfaitement fiable et reproductible permettant de pallier les difficultés précitées relatives aux microparticules, telles que notamment les particules de taille submicronique, notamment en vue d'améliorer l'efficacité et/ou la biodisponibilité des ingrédients actifs constitués par lesdites particules submicroniques.

## OBJET DE L'INVENTION

Suivant l'invention on préconise une nouvelle solution technique pour résoudre le problème de la filtration de fluides gazeux en vue de retenir, à des fins séparatives, analytiques et/ou préparatives, des produits gazeux ou de fines particules.

Cette nouvelle solution technique met en oeuvre l'utilisation d'un matériau cryodesséché ou lyophilisé et comprimé en tant qu'élément poreux de filtration. Elle s'appuie sur le fait que la cryodessiccation ou lyophilisation (i.e. séchage par le froid, opération qui implique la congélation d'une préparation contenant un liquide de solvatation, dilution ou dispersion, notamment à une température de - 40°C et/ou - 80°C, puis l'évaporation dudit liquide congelé par sublimation sous vide), conduit à des produits poreux ayant une surface spécifique (exprimé notamment en $cm^2/cm^3$) relativement importante et pouvant conserver leur texture poreuse jusqu'à ce qu'ils soient mis en contact avec un de leurs solvants.

Selon un des aspects de l'invention, on se propose de fournir un nouveau matériau filtrant, relativement simple à préparer et destiné à être utilisé dans le domaine de la filtration des fluides gazeux afin de retenir essentiellement la majorité des fines particules qu'ils contiennent, notamment quand il s'agit d'aérosols.

Selon un autre aspect de l'invention, on propose un matériau filtrant renfermant dans sa masse au moins un réactif pour fixer et/ou doser les substances gazeuses contenues en tant qu'impuretés ou composants desdits fluides gazeux.

Selon encore un autre aspect de l'invention, on propose un matériau filtrant qui soit soluble dans un solvant organique ou minéral usuel, avantageusement l'eau, en vue de la récupération des matières retenues (notamment des particules submicroniques insolubles dans ledit solvant) et/ou de leur dosage. Inversement, on propose également un matériau filtrant qui soit insoluble dans le solvant sélectif de la matière retenue en vue du dosage de celle-ci.

Suivant un autre aspect de l'invention, on propose un procédé pour la préparation d'un filtre pour fluides gazeux et de l'élément filtrant dudit filtre.

Suivant encore un autre aspect de l'invention, on se propose d'obtenir une forme galénique comprenant au moins un ingrédient actif hydrosoluble ou hydrodispersable.

Suivant un autre aspect de l'invention, on se propose d'obtenir une forme galénique comprenant au moins un ingrédient actif (notamment soluble dans un solvant usuel et insoluble dans les autres solvants classiques) sous forme de microparticules enrobées.

Suivant encore un autre aspect de l'invention, on propose un procédé de préparation des formes galéniques sus-visées utile notamment pour isoler lesdites microparticules et en particulier celles qui ont été enrobées.

## DESCRIPTION DETAILLEE DE L'INVENTION

Selon l'invention on préconise un nouveau filtre pour fluide gazeux qui convient notamment pour retenir les particules contenues ou véhiculées par ledit fluide gazeux et qui ont une taille inférieure ou égale à 5µm, notamment inférieur ou égale à 2,5µm et en particulier les particules ayant une taille inférieure ou égale à 1µm, de préférence une taille comprise entre 1 et 0, 01µm et/ou des produits gazeux contenus dans ledit fluide gazeux que l'on peut épurer. Ce filtre est caractérisé en ce qu'il comporte un élément filtrant lyophilisé et comprimé sous forme de couche poreuse.

Par cryodessiccation ou lyophilisation on entend ici tout séchage par le froid, ce qui implique la congélation d'une préparation liquide ou pateuse puis l'élimination ou évaporation du liquide de solvatation, dilution et/ou dispersion, qui intervient dans la réalisation de ladite préparation, par sublimation, comme indiqué ci-dessus quel que soit ledit liquide.

Plus précisément, le filtre suivant l'invention est caractérisé en ce qu'il comporte un élément filtrant sous forme de couche poreuse qui est constitué d'un matériau solide obtenu à l'état sec (i) par congélation, (ii) par cryodessiccation, puis (iii) par compression de façon à lui conférer une résistance mécanique suffisante pour éviter essentiellement qu'il ne se fissure lors du passage du fluide gazeux à travers son épaisseur.

La compression, qui est effectuée pour améliorer la résistance mécanique du matériau solide filtrant au passage du fluide gazeux à travers son épaisseur, constitue une sorte de frittage présentant une structure poreuse résultant de la structure poreuse obtenue par expansion lors de la cryodessiccation.

Selon une caractéristique de l'invention, l'élément filtrant est constitué d'un matériau solide ayant été comprimé après cryodessiccation suivant un taux de compression compris entre environ 1,1 et environ 5. De façon avantageuse, ledit taux de compression sera compris entre 1,2 et 4, et mieux entre 1,25 et 3,60.

Selon une variante de réalisation, l'élément filtrant qui se présente sous la forme d'une couche poreuse est constitué d'un matériau solide obtenu à l'état sec (a) par congélation, (b) par broyage, (c) par cryodessiccation, puis (d) par compression.

Pour éviter pratiquement toute fissuration de l'élément filtrant lors du passage du fluide gazeux que l'on veut filtrer, on recommande d'incorporer dans la masse du matériau solide constituant ledit élément filtrant un agent de cohésion approprié. Ledit agent de cohésion peut notamment être choisi parmi l'ensemble constitué par les substances plastifiantes. En variante, l'agent de cohésion plastifiant peut être une charge organique notamment polymère qui est susceptible d'être expansée par lyophilisation, et qui est douée de propriétés plastifiantes. Comme exemples de charges plastifiantes suivant l'invention on peut notamment mentionner l'hydroxypropylméthylcellulose, l'alcool polyvinylique et la poudre de lait.

Selon un autre mode préféré de réalisation, la concentration de l'agent de cohésion plastifiant présent dans la masse du matériau solide de l'élément filtrant est comprise entre 1 % et 75 % en poids par rapport au total dudit matériau solide.

Le matériau solide, qui constitue l'élément filtrant du filtre suivant l'invention, comprend essentiellement une ou plusieurs charges minérales ou organiques, inertes, non-réactives vis-à-vis des fluides gazeux devant être filtrés. Parmi les charges qui conviennent, on peut notamment mentionner le verre, la céramique, la silice, le carbone, les dérivés cellulosiques, les résines et polymères organiques notamment les matériaux polyacryliques, polyméthacryliques, polyvinylpyrrolidones, polystyrènes, polyuréthanes et leurs mélanges. Ces charges peuvent être sous forme granulaire, particulaire ou sous forme de fibres. Bien entendu, les mélanges de particules et de fibres confèrent à l'élément filtrant une meilleure cohésion vis-à-vis des fissurations, les fibres intervenant alors en tant que charge et moyens de cohésion.

Parmi les charges minérales, on peut ainsi utiliser les fibres de verre, les fibres de céramique, les particules de carbone expansées et la silice. On peut également utiliser les particules de métaux, de sels métalliques et d'oxydes métalliques, quand ces substances sont inertes vis-à-vis des fluides gazeux que l'on veut utiliser dès lors que, comme illustré plus loin,lesdites substances peuvent intervenir en tant que réactifs notamment dans le dosage ou la détection de produits gazeux contenus dans certains fluides gazeux.

Parmi les charges organiques qui conviennent on peut ainsi utiliser les substances polymériques sous forme de particules ou de fibres.

D'une manière générale, on préfère que la charge, qui va être lyophilisée et comprimée pour constituer la masse ou matrice de l'élément filtrant, soit essentiellement sous forme de particules avec, le cas échéant, une faible teneur en fibres pour la cohésion dudit élément filtrant.

Suivant un mode avantageux de mise en oeuvre de l'invention, l'élément filtrant est constitué d'un matériau solide soluble ou dispersable dans un solvant choisi parmi l'ensemble comprenant l'eau, les solvants minéraux, les solvants organiques et leurs mélanges.

De façon appropriée, on préfère plus particulièrement les charges qui sont solubles ou microdispersables dans l'eau, à savoir : les matériaux polysaccharidiques tels que le dextrane, la carboxyméthylcellulose, l'hydroxypropylcellulose, les matériaux mono- ou disaccharidiques tels que le glucose, le lactose, le maltose, le saccharose, les matériaux polyvinyliques tels que l'alcool polyvinylique, les matériaux polyacryliques tels que les sels ou esters polyacrylates et polyméthacrylates, les polyvinylpyrrolidones, et leurs mélanges, notamment la poudre de lait précitée, qui est un produit renfermant notamment du lactose, de la caséine et d'autres substances protéiniques et polypeptidiques, et les extraits de lait. En variante, on peut utiliser une charge insoluble dans l'eau mais soluble dans un solvant organique usuel approprié.

L'élément filtrant suivant l'invention, peut également comporter dans sa masse un ou plusieurs réactifs notamment pour analyse de mélanges de gaz.

Il suffit par exemple d'introduire dans le matériau de l'élément filtrant un corps basique pour qu'il retienne les traces acides dans le mélange analysé (notamment les oxydes de soufre et les oxydes d'azote, les vapeurs d'HCl, etc ...). On peut aussi y introduire des métaux divisés permettant de mesurer le rapport $CO/CO_2$ par dosage du méthane résultant de la transformation catalytique du CO à l'aide d'un détecteur à capture d'électrons.

L'élément filtrant suivant l'invention, qui est obtenu par congélation, broyage (pour donner des granulats d'une granulométrie de l'ordre de 1 à 3 mm environ), cryodessiccation puis compression offre l'avantage d'être relativement léger et de pouvoir de façon remarquable être dissous ou dispersé aisément dans de très faibles quantités de solvants appropriés après passage du fluide gazeux à filtrer.

Ledit élément filtrant lyophilisé et comprimé est placé sur le parcours d'un fluide gazeux circulant contenant, par exemple, des particules de taille micronique ou submicronique, par exemple un échantillon d'air atmosphérique prélevé dans des régions à pollution urbaine ou industrielle élevée. Un certain volume d'air correspondant à une masse déterminée de gaz aux conditions normales de température et de pression, éventuellement déshydraté par passage dans un "denuder", à adsorption ou à porosité sélective, est conduit à passer au travers d'une couche plus ou moins épaisse dudit élément filtrant. Du fait de la très grande porosité de ce matériau, l'air y circule très facilement, sans perte de charge appréciable. De plus, la structure très fine du matériau provoque la capture des éléments particulaires solides qui peuvent s'y déposer par impact, mouvement brownien, adsorption ou par suite de forces électrostatiques. A ce titre, le matériau lyophilisé et comprimé suivant l'invention se comporte comme un filtre à haute efficacité.

Le matériau suivant l'invention qui constitue l'élément filtrant convient parfaitement pour la filtration de particules microniques et submicroniques, c'est-à-dire ayant une granulométrie comprise entre 5 et 0,01$\mu$m, notamment comprise entre 2,5 et 0,01$\mu$m et mieux comprise entre 1 et 0,01$\mu$m, véhiculées par un fluide gazeux.

La lyophilisation ou cryodessiccation, qui intervient dans la préparation de ce matériau, est réalisée selon une méthode connue en soi, par exemple suivant une technique décrite par L.R. REY et al., "Traité de lyophilisation", Hermann éditeur, Paris 1961, pages 1-411; L.R. REY, Experientia 21, pages 241-246, (1965); L.R. REY, Proc. Roy. Soc. B, London 191, pages 9-19, (1975); US-A-4 616 047; US-A-4 178 695; GB-A-1 328 641; US-A-4 490 407; et, US-A-3 939 260.

Suivant l'invention, le matériau filtrant est également utile pour l'obtention et la séparation de particules microniques et mieux submicroniques d'ingrédients actifs pharmaceutiques. Ces particules d'ingrédients actifs peuvent être enrobées au moyen d'un revêtement soluble ou insoluble dans l'eau ; parmi les revêtements insolubles dans l'eau, qui conviennent, on peut mentionner les revêtements liposolubles destinés à un délitage au niveau des intestins.

Le procédé de préparation de l'élément filtrant selon l'invention est caractérisé en ce que l'on soumet une préparation sous forme liquide ou pâteuse d'une substance polymère et d'un liquide de solvatation, dilution ou dispersion à une congélation à une température de - 40°C à - 80°C, à un broyage du produit solide congelé ainsi obtenu, à une cryodessiccation du broyat résultant pour sublimer le liquide de solvatation, puis on soumet le produit cryodesséché ainsi obtenu à une compression à un taux compris entre 1,1 et 5.

On préconise également selon l'invention, en tant que produit industriel nouveau, une forme nouvelle galénique ou composition, ladite forme galénique comprenant en association (i) une matrice physiologiquement acceptable et (ii) au moins un ingrédient actif sous forme particulaire et choisi parmi les principes actifs thérapeutiques et cosmétiques, et étant caractérisée en ce que :

A) la matrice physiologiquement acceptable est un matériau solide et poreux qui a été obtenu à l'état sec par congélation, cryodessiccation et compression pour conférer audit matériau une résistance mécanique suffisante pour éviter essentiellement qu'il ne se fissure lors du passage d'un fluide gazeux à travers son épaisseur ; et,

B) l'ingrédient actif associé à ladite matrice est constitué essentiellement par des microparticules ayant une granulométrie moyenne comprise entre 5$\mu$m et 0,01$\mu$m, et choisi parmi les substances qui sont (1) solubles dans l'eau, (2) insolubles dans l'eau, ou (3) enrobées.

On vise donc ici une forme galénique ou composition où le principe actif est sous forme de microparticules et est soit soluble soit insoluble dans l'eau, d'une part, ou est enrobé quelle que soit sa solubilité dans l'eau, d'autre part.

On préconise aussi un procédé de préparation d'une telle forme galénique ou composition, ledit procédé étant caractérisé en ce qu'il comprend les étapes suivantes :

1°) obtention d'une préparation de l'ingrédient actif dans un milieu liquide, ledit ingrédient actif étant soit soluble soit insoluble dans ledit milieu liquide et sa concentration dans ledit milieu liquide étant inférieure ou égale à 10 % (p/v) ;

2°) nébulisation de la prération résultante pour obtenir un aérosol entraîné dans un courant de fluide gazeux porteur ;

3°) évaporation du milieu liquide de l'aérosol, de façon que les microparticules qui résultent du séchage dudit aérosol soient véhiculées par ledit fluide gazeux porteur ;

4°) filtration dudit fluide gazeux porteur contenant l'ingrédient actif essentiellement sous forme de microparticules au moyen d'un filtre comprenant, comme élément filtrant, un matériau solide et poreux qui a été obtenu à l'état sec par congélation, cryodessiccation et compression pour conférer audit matériau une résistance mécanique suffisante pour éviter essentiellement qu'il ne se fissure lors du passage d'un fluide gazeux à travers son épaisseur, pour recueillir sur l'élément filtrant lesdites microparticules.

La forme ou composition galénique suivant l'invention, qui est utile en thérapeutique et en cosmétique, contient des microparticules (soit solubles soit insolubles dans l'eau d'une part, soit enrobées quelle que soit leur hydrosolubilité, d'autre part) ayant une granulométrie moyenne comprise entre 5μm et 0,01μm. Parmi ces particules on préfère celles qui ont une granulométrie moyenne inférieure ou égale à 2,5μm et mieux inférieure ou égale à 1μm.

La matrice de la forme galénique est obtenue à partir de l'élément filtrant sus-visé. Cet élément filtrant qui se présente sous forme de couche poreuse est constitué d'un matériau solide obtenu à l'état sec (i) par congélation, (il) par cryodessiccation, puis (iii) par compression de façon à lui conférer une résistance mécanique suffisante pour éviter essentiellement qu'il se ne fissure lors du passage d'un fluide gazeux à travers son épaisseur.

Le taux de compression utile pour la préparation de la forme galénique est compris comme indiqué ci-dessus entre environ 1,1 et environ 5. De façon avantageuse, ledit taux de compression sera compris entre 1,2 et 4, et mieux encore entre 1,25 et 3,60.

L'enrobage des microparticules de l'ingrédient actif est effectué afin de modifier les conditions de délitage dudit ingrédient actif dans l'organisme, en particulier par voie orale. Il permet notamment de pallier des propriétés dites négatives, telles que arrière-goût amer, amertume prononcée ou solubilisation trop rapide de l'ingrédient actif dans les conditions normales d'administration. L'enrobage permet aussi, en formant une enveloppe, couche ou pellicule essentiellement continue autour desdites microparticules, de protéger l'ingrédient actif. Cette enveloppe peut demeurer intacte jusqu'à ce qu'elle soit en contact avec ou en circulation dans un fluide corporel spécifique ou approprié. L'enrobage peut ainsi disparaître par dégradation ou dissolution à un niveau déterminé du tractus digestif afin de libérer l'ingrédient actif, par exemple au niveau de la muqueuse intestinale dans le cas d'un enrobage gastro- ou acido-résistant. L'enrobage peut enfin servir à favoriser l'assimilation des microparticules dans l'organisme, suivant un effet "masque" ou un effet "liposome", selon un mode préféré de l'invention, surtout lorsque la taille ou granulométrie moyenne des particules qui résultent de l'enrobage des microparticules est de l'ordre du micromètre ou mieux inférieure au micromètre.

Pour la mise en oeuvre du procédé de préparation de la forme galénique suivant l'invention, on préfère que la teneur en ingrédient actif de la solution ou dispersion dans l'eau ou le solvant organique de l'étape 1°) soit inférieure ou égale à 5 % (p/v).

La nébulisation, mise en oeuvre à l'étape 2°), est effectuée de façon à obtenir des microparticules liquides d'aérosol ayant un diamètre moyen approprié pour donner après évaporation du solvant de dissolution ou de dispersion des microparticules solides ayant une granulométrie moyenne comprise entre 5μm et 0,01μm, de préférence une granulométrie moyenne inférieure ou égale à 2,5μm, et mieux une granulométrie moyenne inférieure ou égale à 1μm.

Le fluide gazeux porteur qui convient suivant l'invention est un gaz inerte vis-à-vis de l'ingrédient actif et de la matrice constituée par l'élément filtrant, par exemple l'azote, l'argon et, le cas échéant, l'air desséché.

L'évaporation de l'étape 3°) est effectuée à une température appropriée de façon à ne pas détériorer l'ingrédient actif, notamment à une température comprise comprise entre 25° et 90° C. De façon avantageuse, on opérera à une température entre 30° et 65° C. Le cas échéant, on pourra utiliser un gradient de température croissant ou décroissant.

La filtration de l'étape 4°) est effectuée avec une perte de charge comprise entre environ 80 et environ 10 000 Pa. La perte de charge, qui est notamment fonction de l'épassieur de l'élément filtrant, de son taux de compression (rapport de l'épaisseur du matériau avant compression à l'épaisseur dudit matériau après compression) et de la porosité résultante, ainsi que de la pression du fluide gazeux à filtrer, est généralement comprise entre 1 cm $H_2O$ (environ 98,06 Pa) et 100 cm $H_2O$ (environ 9806 Pa), soit plus précisément entre 2 cm $H_2O$ (environ 196,1 Pa) pour une épaisseur de 8 mm de matériau filtrant à un taux de compression de 1,25 pour un fluide gazeux ayant un débit de 300 l/min rapporté aux conditions normales de température et de pression, et 76 cm $H_2O$ (soit environ 7452,5 Pa) pour une épaisseur de 14 mm de matériau filtrant à un taux de compression de 3,6 pour un fluide gazeux ayant un débit de 4 l/min rapporté aux conditions normales de température et de pression.

Après la filtration de l'étape 4°), le procédé de l'invention comprend en outre pour l'obtention d'une forme galénique sensiblement homogène une étape 5°) dite de malaxage, suivant laquelle on mélange intimement l'élément filtrant et les microparticules qui ont été retenues par ledit élément filtrant à l'étape 4°). En effet, en raison du mode de filtration, la plus grande partie des microparticules retenues se trouve rassemblée dans l'épaisseur de l'élément filtrant au voisinage de la face d'entrée dudit élément filtrant recevant le fluide gazeux contenant les microparticules solides que l'on veut recueillir.

En bref, pour les principes actifs thérapeutiques et cosmétiques, qui sont insolubles dans l'eau, ladite forme galénique est caractérisée en ce que :

(A) la matrice physiologiquement acceptable est un matériau solide et poreux qui a été obtenu à l'état sec par congélation, cryodessiccation et compression pour conférer audit matériau une résistance mécanique suffisante pour éviter essentiellement qu'il ne se fissure lors du passage d'un fluide gazeux à travers son épaisseur ; et,

(B) l'ingrédient actif associé à ladite matrice est insoluble dans l'eau et est constitué essentiellement par des microparticules ayant une granulométrie moyenne comprise entre 5$\mu$m et 0,01$\mu$m.

Ledit procédé de préparation comprend alors les étapes suivantes :

1°) obtention d'une solution dans un solvant organique dudit ingrédient actif insoluble dans l'eau, de façon que la concentration dudit ingrédient actif dans ladite solution soit inférieure ou égale à 10 % (poids / volume) ;

2°) nébulisation de ladite solution ainsi obtenue, sous forme d'aérosol liquide entraîné dans un courant de fluide gazeux porteur ;

3°) évaporation du solvant de l'ingrédient actif dans ledit courant de fluide gazeux porteur, de façon que les microparticules solides résultant de la dessiccation des microparticules liquides de l'aérosol soient véhiculées par ledit fluide gazeux porteur ;

4°) filtration dudit fluide gazeux porteur contenant l'ingrédient actif essentiellement sous forme de microparticules ayant une granulométrie moyenne comprise entre 5$\mu$m et 0,01$\mu$m, d'une part, et le solvant dudit ingrédient actif sous forme de vapeur, d'autre part, au moyen d'un filtre comprenant, comme élément filtrant, un matériau solide, poreux et hydrosoluble qui a été obtenu à l'état sec par congélation, cryodessiccation et compression pour conférer audit matériau une résistance mécanique suffisante pour éviter essentiellement qu'il ne se fissure lors du passage d'un fluide gazeux à travers son épaisseur.

Selon encore un autre aspect de l'invention on propose une utilisation de ce procédé de préparation pour recueillir les microparticules de l'ingrédient actif insoluble dans l'eau, suivant laquelle on dissout dans l'eau, la matrice qui lui est associée et qui a été utilisée comme élément filtrant au cours de la filtration de l'étape 4°).

Pour les principes actifs thérapeutiques et cosmétiques, qui sont hydrosolubles ou enrobés, la forme galénique est caractérisée en ce que :

A) la matrice physiologiquement acceptable est un matériau solide et poreux qui a été obtenu à l'état sec par congélation, cryodessiccation et compression pour conférer audit matériau une résistance mécanique suffisante pour éviter essentiellement qu'il ne se fissure lors du passage d'un fluide gazeux à travers son épaisseur ; et,

B) l'ingrédient actif associé à ladite matrice est constitué essentiellement par des microparticules ayant une granulométrie moyenne comprise entre 5$\mu$m et 0,01$\mu$m, et choisi parmi les substances qui sont (1) solubles dans l'eau, ou (2) enrobées.

Quand l'ingrédient actif est hydrosoluble, ledit procédé de préparation de la forme galénique comprend les étapes suivantes :

1°) obtention d'une solution dudit ingrédient actif dans l'eau, de façon que la concentration dudit ingrédient actif dans ladite solution soit inférieure ou égale à 10 % (p/v) ;

2°) nébulisation de ladite solution, ainsi obtenue, sous forme d'aérosol liquide entraîné dans un courant de fluide gazeux porteur ;

3°) évaporation de l'eau, solvant de l'ingrédient actif, dans ledit courant de fluide gazeux porteur, de façon que les microparticules solides résultant de la dessiccation des microparticules liquides de l'aérosol soient véhiculées par ledit fluide gazeux porteur ;

4°) filtration dudit fluide gazeux porteur contenant l'ingrédient actif essentiellement sous forme de microparticules ayant une granulométrie moyenne comprise entre 5$\mu$m et 0,01$\mu$m, d'une part, et le solvant dudit ingrédient actif sous forme de vapeur, d'autre part, au moyen d'un filtre comprenant, comme élément filtrant, un matériau solide et poreux qui a été obtenu à l'état sec par congélation, cryodessiccation et compression pour conférer audit matériau une résistance mécanique suffisante pour éviter essentiellement qu'il ne se fissure lors du passage d'un fluide gazeux à travers son épaisseur.

Quand l'ingrédient actif est sous la forme de microparticules enrobées, ledit procédé est caractérisé en

EP 0 298 864 B1

ce qu'uil comprend les étapes suivantes :

1°) obtention d'une suspension de microparticules de l'ingrédient actif dans un milieu liquide contenant un matériau d'enrobage dissous, ledit ingrédient actif étant insoluble dans ledit milieu liquide et sa concentration dans ledit milieu liquide étant inférieure ou égale à 10 % (p/v) ;

2°) nébulisation de la suspension résultante pour obtenir un aérosol entraîné dans un courant de fluide gazeux porteur ;

3°) évaporation du milieu liquide de l'aérosol, de façon que les microparticules enrobées qui résultent du séchage dudit aérosol soient véhiculées par ledit fluide gazeux porteur ;

4°) filtration dudit fluide gazeux porteur contenant l'ingrédient actif essentiellement sous forme de microparticules enrobées par le matériau d'enrobage au moyen d'un filtre comprenant, comme élément filtrant, un matériau solide et poreux qui a été obtenu à l'état sec par congélation, cryodessiccation et compression pour conférer audit matériau une résistance mécanique suffisante pour éviter essentiellement qu'il ne se fissure lors du passage d'un fluide gazeux à travers son épaisseur, pour recueillir sur l'élément filtrant lesdites microparticules enrobées.

Pour l'obtention de microparticules enrobées, on préconise suivant l'invention un procédé qui comprend :

- la nébulisation d'une solution de l'ingrédient actif dans l'eau ou un solvant organique,
- le séchage de l'aérosol résultant entraîné dans un fluide gazeux porteur ou vecteur,
- la filtration dudit fluide gazeux au moyen d'un élément filtrant obtenu par congélation, broyage, cryodessiccation puis compression,
- la dissolution dudit élément filtrant dans un solvant où le matériau d'enrobage est soluble, d'une part, et les microparticules d'ingrédient sont insolubles, d'autre part,
- la nébulisation de la dispersion ou suspension résultante,
- le séchage de l'aérosol entraîné dans un fluide gazeux porteur comme indiqué ci-dessus; et,
- la filtration sur un élément filtrant obtenu par congélation, broyage, cryodessiccation puis compression.

Selon le meilleur mode de mise en oeuvre de l'invention, le procédé de préparation de microparticules enrobées est caractérisé en ce qu'il comprend les étapes suivantes :

(a) obtention d'une solution de l'ingrédient actif dans un premier solvant, de façon que la concentration dudit ingrédient actif dans ledit premier solvant soit inférieure ou égale à 10 % (p/v) et de préférence inférieure ou égale à 5 % (p/v) ;

(b) nébulisation de ladite solution, ainsi obtenue, sous forme d'aérosol liquide entraîné dans un courant de fluide gazeux porteur ;

(c) évaporation du solvant de l'ingrédient actif dans ledit courant de fluide gazeux porteur, de façon que les microparticules solides résultant de la dessiccation des microparticules liquides de l'aérosol soient véhiculées par ledit fluide gazeux porteur ;

(d) filtration dudit fluide gazeux porteur contenant l'ingrédient actif essentiellement sous forme de microparticules ayant une granulométrie moyenne comprise entre $5\mu m$ et $0,01\mu m$, d'une part, et le solvant dudit ingrédient actif sous forme de vapeur, d'autre part, au moyen d'un filtre comprenant, comme élément filtrant, un matériau solide et poreux qui a été obtenu à l'état sec par congélation, broyage, cryodessiccation et compression pour conférer audit matériau une résistance mécanique suffisante pour éviter essentiellement qu'il ne se fissure lors du passage d'un fluide gazeux à travers son épaisseur ;

(e) solubilisation de l'élément filtrant utilisé à l'étape (d) dans un second solvant dans lequel (i) l'ingrédient actif est insoluble, et (ii) le matériau d'enrobage est soluble, afin d'obtenir un milieu liquide constitué par ledit second solvant où est dissous le matériau d'enrobage et où sont dispersées les microparticules à enrober ;

(f) nébulisation de la suspension résultante pour obtenir un aérosol entraîné dans un courant de fluide gazeux porteur ;

(g) évaporation du solvant du milieu liquide pour le séchage de l'aérosol, de façon que les microparticules enrobées qui résultent du séchage dudit aérosol soient véhiculées par ledit fluide gazeux porteur ;

(h) filtration dudit fluide gazeux porteur contenant les particules d'ingrédient actif enrobées au moyen d'un filtre comprenant, comme élément filtrant, un matériau solide et poreux qui a été obtenu à l'état sec par congélation, broyage, cryodessiccation et compression pour conférer audit matériau une résistance mécanique suffisante pour éviter essentiellement qu'il ne se fissure lors du passage d'un fluide gazeux à travers son épaisseur.

Le matériau d'enrobage qui convient suivant l'invention est une substance organique filmogène, soluble dans un solvant usuel spécifique et insoluble dans les autres solvants usuels non spécifiques. Le matériau

8

d'enrobage est en général de nature polymérique. Conviennent notamment les polymères et copolymères dérivés de l'acide acrylique tels que les acides polyacryliques, les acides polyméthacryliques, leurs sels et leurs esters, les dérivés cellulosiques, les glycérides, les polyglycérides d'acides gras, les lécithines, les huiles hydrogénées, les polyoxyalkylèneglycols tels les polyoxyéthylèneglycols et polyoxypropylèneglycols, des polyacides tels que les acides polylactiques et polygluconiques, et leurs mélanges.

Conviennent d'une manière générale comme matériau d'enrobage, les cires, graisses et autres moyens d'enrobage physiologiquement acceptables qui sont de façon typique utilisés ou utilisables en galénique, par exemple un mélange pondéral 1/1 d'éthylcellulose (produit commercialisé sous le nom de marque "AQUACOAT" par la société dite SEPPIC) et d'hydroxypropylméthylcellulose (produit commercialisé sous le nom de "PHARMACOAT 603" par la société dite SEPPIC).

Les microparticules suivant l'invention qu'elles soient enrobées ou non-enrobées peuvent, le cas échéant, être récupérées par dissolution de la matrice qui a été utilisée comme élément filtrant au cours de la filtration de l'étape 4°). Cette dissolution est réalisée dans un solvant approprié dans lequel les dites microparticules, le cas échéant enrobées, sont insolubles.

D'autres avantages et caractéristiques de l'invention seront mieux compris à la lecture de la description qui va suivre d'exemples de réalisation et de dessins où :

- la figure 1 représente de façon schématique une vue en coupe d'un filtre suivant l'invention comportant un élément filtrant lyophilisé et comprimé,
- la figure 2 représente le schéma d'une installation permettant d'apprécier l'efficacité d'un filtre, d'une part, et de doser les particules véhiculées par un fluide gazeux, d'autre part, et
- la figure 3 est la reproduction d'une photographie réalisée au microscope montrant les particules submicroniques retenues par l'élément filtrant suivant l'invention.

Bien entendu, l'ensemble de ces éléments est donné à titre d'illustration et n'est nullement limitatif.

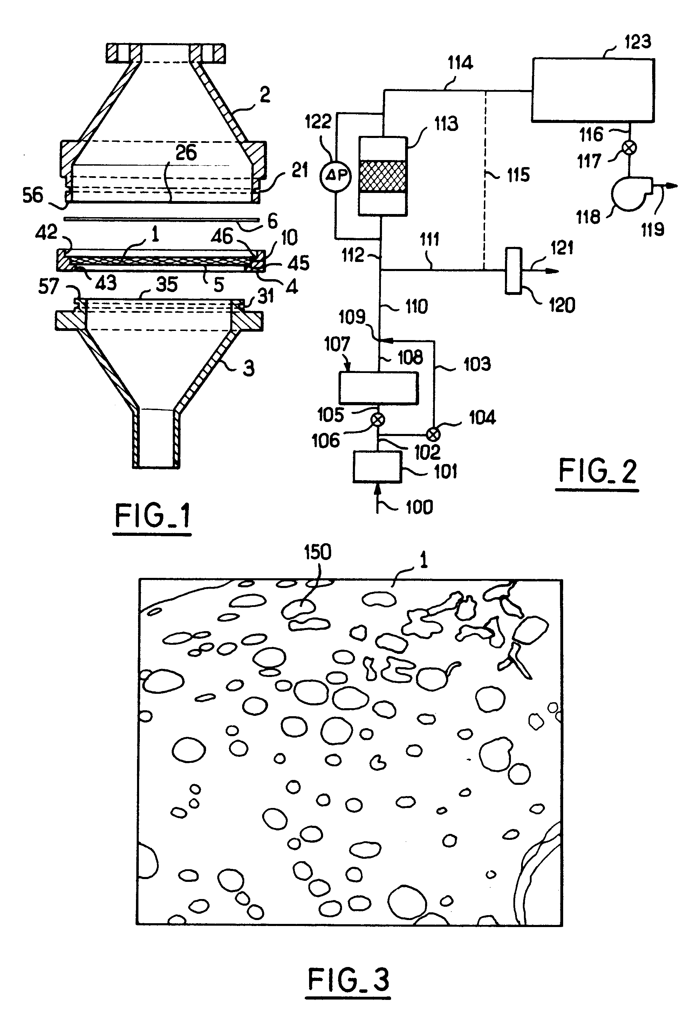

Le meilleur mode de mise en oeuvre de l'invention, en ce qui concerne la préparation de l'élément filtrant et son efficacité, est représenté par la figure 1 et les exemples 2-3.

Suivant la figure 1, le filtre conforme à l'invention comprend un élément filtrant 1 sensiblement perpendiculaire à la direction d'un fluide gazeux à filtrer et situé entre deux conduits 2, 3 parallèlement, d'une part, à l'extrémité de sortie 26 du conduit d'arrivée 2 dudit fluide et, d'autre part, à l'extrémité d'entrée 35 du conduit de sortie 3 dudit fluide, ce filtre étant caractérisé en ce que ledit élément filtrant 1, qui est constitué d'un matériau solide obtenu à l'état sec sous forme poreuse par congélation, cryodessication puis compression, est disposé entre deux plaques perforées 5, 6, notamment des grilles, l'une 6 étant destinée à s'adapter au rebord 56 de l'extrémité 26 dudit conduit 2 d'arrivée, l'autre 5 étant destinée à s'adapter au rebord 57 de l'extrémité 35 dudit conduit 3 de sortie, lesdits conduits 2, 3 étant agencés de telle façon que lesdits rebords 56, 57 coopèrent en tant que machoires périphériques maintenant en place ledit élément filtrant 1 entre lesdites plaques perforées 5, 6 logées entre lesdites extrémités 26, 35, d'une part, et que la tranche latérale 10 de l'élément filtrant 1 devienne au moment de l'utilisation imperméable audit fluide gazeux, d'autre part.

Plus précisément ce filtre comprend trois pièces : le conduit d'arrivée 2, le conduit de sortie 3 et le support 4 de l'élément filtrant 1 qui est un disque disposé entre les plaques perforées 5 et 6, qui sont ici, de façon avantageuse, des grilles ayant une ouverture de maille très supérieure au diamètre des particules microniques et submicroniques devant être retenues.

La grille supérieure 6 est destinée à reposer sur la platine annulaire 46 du support 4 et à venir en contact du rebord 56 du conduit 2.

La grille inférieure 5 est destinée à s'appuyer sur la platine annulaire 45 du support 4, d'une part, et sur le rebord 57 du conduit 3, d'autre part.

Comme moyen d'étanchéité latérale les conduits 2 et 3 comportent chacun une gorge 21 et respectivement 31 au voisinage des extrémités 26 et respectivement 35, lesdites gorges recevant chacune un joint torique (non représenté ici), les parois latérales 42 et 43 du support 4 viennent en contact intime avec les parois latérales extérieures correspondantes des conduits 2 et respectivement 3, d'une part, et les joints toriques logés dans les gorges 21 et respectivement 31, d'autre part, lors du montage du filtre pour sa mise en service.

Par ailleurs, les conduits 2 et 3 peuvent en outre comporter un ou plusieurs moyens (non représentés ici) leur permettant de devenir solidaires l'un de l'autre.

## EXEMPLE 1

On prépare une solution aqueuse à 7,5 % en poids de Dextran 70 dans de l'eau. La solution clarifiée est versée sur une épaisseur de 10 mm dans des récipients cylindriques (couvercles de boîtes de Petri) d'un diamètre de 60 mm, dont les parois ont été préalablement siliconées, puis congelée à - 50°C de façon progressive.

Le matériau congelé est ensuite lyophilisé sous vide poussé (0,01 Torr, soit environ 1,333 Pa) puis ramené à la pression atmosphérique et à la température ambiante par rupture du vide sur un gaz neutre et sec (azote).

Le matériau lyophilisé ainsi obtenu, que se présente alors sous la forme d'un disque cylindrique très léger mais relativement résistant, est placé dans le support de filtre 4 d'un élément standard de filtration du type de ceux utilisés par le NILU (Institut Norvégien pour la Recherche sur l'Air, situé à Oslo) et modifié comme représenté à la figure 1 avec des grilles 5 et 6 pour le maintien de l'élément filtrant 1 et des gorges 21 et 31 pourvues chacune d'un joint torique.

L'appareil porte-filtre est inséré dans une chaîne d'analyse entre un générateur de particules calibrées, du type TSI, Tri-Jet Aerosol Generator Model 3460, et un compteur de particules à laser (PMS LAS-XCRT). Une petite pompe de circulation aspire alors, au travers du filtre, 40 ml/s d'un air contenant une population stable de particules de latex de deux dimensions : $0,176\mu m$ et $0,62\mu m$, en provenance du générateur.

Le schéma de cette chaîne est illustré par la figure 2. On injecte de l'air comprimé en 100 que l'on filtre selon une méthode connue en soi au moyen d'un filtre classique 101 muni d'un dispositif permettant d'éliminer substantiellement l'eau contenue dans ledit air comprimé, si cela est nécessaire. Le flux d'air filtré et déshydraté 102, qui sort du filtre 101, est divisé en deux courants : un courant de dilution passant par la canalisation 103 pourvue d'une pompe 104 de régulation, et un courant passant par la canalisation 105 munie d'une pompe 106 débouchant dans un générateur d'aérosol 107. Le courant chargé en aérosol 108 est dilué en 109 par le courant provenant de la canalisation 103. Le fluide gazeux résultant suit la conduite 110 et se répartit à nouveau en deux courants : un courant 111 dirigé vers un filtre classique 120 avec une sortie libre 121 ou vers une dérivation éventuelle 115 débouchant dans la conduite 114, et un courant dirigé par la conduite 112 vers une section 113 comportant un dispositif de filtration selon l'invention tel que représenté à la figure 1. Une dérivation pourvue d'un manomètre 122 est située en regard de la section 113. Le fluide gazeux filtré selon l'invention est dirigé suivant la conduite 114 vers un compteur optique de particules 123 (notamment un détecteur laser) puis suivant une conduite 116 munie d'un dispositif de contrôle de débit 117 et d'une pompe 118 vers la sortie 119.

Avec une telle chaîne, le comptage des particules dans l'air sortant, intégré par périodes d'une minute, indique que la capture des particules est :

- de 90 % pour la taille de $0,62\mu m$,
- \> 95 % pour la taille de $0,176\mu m$.

Ces résultats, bien que très satisfaisants pour des particules submicroniques, indiquent cependant que le filtrage n'est pas total. Ceci peut s'expliquer par le fait que le matériau lyophilisé à base de Dextran pur manque d'élasticité et que, de ce fait, des microfissures apparaissent dans sa masse tant au cours du séchage que lors de la mise sous tension à l'intérieur du portefiltre par l'intermédiaire des joints toriques.

### EXEMPLE 2

On prépare une solution aqueuse contenant Dextran 70 (4%), Pharmacoat 603 (ou hydroxypropylméthylcellulose) (4 %) et saccharose (0,0125 %). La solution clarifiée est versée dans des boîtes métalliques rectangulaires, préalablement siliconées, sur une épaisseur de 10 mm puis congelée à - 50°C comme précédemment.

A la température de - 50°C, la solution congelée est démoulée puis les plaques résultantes sont broyées à froid à l'aide d'un broyeur à marteaux jusqu'à obtention d'un granulé homogène d'environ 2 à 3 mm de taille de grains, qui est alors lyophilisé ou cryodesséché comme décrit ci-dessus.

Après lyophilisation et mise sous gaz neutre, le granulé lyophilisé est placé dans un porte-filtre cylindrique fermé à sa partie inférieure par une grille fine reposant sur un diaphragme circulaire.

On verse ensuite dans le porte-filtre le granulé lyophilisé sur une hauteur totale de 50 mm, alternativement 25 mm, puis on place l'ensemble sur une table support permettant l'adaptation d'un piston cylindrique qui est ajusté à la partie haute du portefiltre. On procède alors à une compression contrôlée de la poudre jusqu'à réduction de l'épaisseur totale dans un rapport déterminé.

On obtient de la sorte un élément filtrant lyophilisé "fritté" sous forme d'un disque compact très homogène et sans fissuration.

L'ensemble de l'élément filtrant et du portefiltre est alors placé dans un appareillage analogue à celui utilisé pour l'exemple 1, entre une source de particules calibrées ($0,017$ - $0,350$ - $1,091\mu m$) et un détecteur laser 123 de particules sortantes (figure 2). On procède ensuite à une injection continue d'air chargé en aérosol de particules de latex (4 l/min) et l'on analyse l'air sortant pour évaluer l'importance de la filtration.

Le tableau suivant donne les résultats de la capture pour les différentes tailles des particules en fonction du taux de compression.

**Efficacité de capture de microparticules par un filtre lyophilisé**

| | Epaisseur (cm) | Rapport de compression | Densité (g/cm$^3$) | Perte de charge (Pa) | Efficacité de capture | | |
|---|---|---|---|---|---|---|---|
| | | | | | $d_p=1,091\,\mu m$ | $d_p=0,350\,\mu m$ | $d_p=0,107\,\mu m$ |
| Dextran 70 (4 %) + | 1,96 | 2,6 | 0,10 | 3334 | 100,0 | 99,99 | 99,70 |
| Pharmacoat 603 (4 %) | 1,40 | 3,6 | 0,14 | 7452,5 | 100,0 | 99,99 | 99,93 |
| +Saccharose (0,0125 %) | 1,27 | 2,0 | 0,08 | 2745,6 | 99,99 | 99,86 | 93,62 |

Ces résultats mettent en évidence, qu'il est possible, en utilisant rationnellement un matériau lyophilisé plus "plastique et rassemblé en élément filtrant "fritté" par compression contrôlée, de réaliser un filtre pratiquement absolu retenant la quasi totalité des particules les plus fines dans un courant gazeux vecteur d'aérosol submicronique.

## EXEMPLE 3

Un élément filtrant lyophilisé compacté est réalisé comme décrit précédemment à l'exemple 2 à partir d'une solution aqueuse contenant Dextran 70 (4 %), Pharmacoat 603 (4 %), saccharose (0,0125 %). L'élément filtrant est placé sur un support dans un filtre à la sortie du courant de microparticules émis par un générateur de microparticules selon l'invention tel que cité précédemment. Dans le cas du présent exemple, les microparticules sont préparées à partir d'une solution à 2,5 % (p/v) de nifédipine [ester diméthylique de l'acide 1,4-dihydro-2,6-dimethyl-4-(2-nitro-phényl)-3,5-pyridine-carboxylique] dans le chloroforme injectée dans un courant d'azote dans les nébulisateurs d'un générateur de microparticules sous une pression de 4 bars à la température de 25°C et à un rythme de 300 l (normal) de gaz/min. L'aérosol ainsi formé est séché dans l'appareil à la température de 30°C et les microparticules formées sont recueillies sur un élément filtrant fritté identique à celui décrit dans l'exemple 2, d'une épaisseur finale de 8 mm après une légère compression à partir d'une couche initiale de granulé de 10 mm.

Dans ces conditions, la perte de charge au niveau du filtre est insignifiante (2 cm d'eau, i.e. 196,1 Pa) et l'on constate que l'intégralité des particules se trouve piégée dans les trois premiers millimètres de l'élément filtrant, qui devient jaune, et où l'on peut accumuler, en dix minutes, 4,5 g de produit actif pour un poids total de matériel filtrant de 115 g.

Il suffit alors de retirer l'élément filtrant et de l'homogénéiser pour disposer d'une matière première intermédiaire élaborée ayant une teneur de 3,9 % en nifédipine sous forme submicronique.

La figure 3 représente schématiquement une photographie prise au microscope (G = 10 000) après filtration suivant l'invention de particules submicroniques de nifédipine conformément à l'exemple 3. L'élément filtrant 1 comprend à sa surface des particules 150 de nifédipine. Sous l'impact, lesdites particules que l'on voit à la surface de l'élément filtrant 1 ont partiellement pénétré dans la masse du matériau de l'élément filtrant. D'une manière générale, les particules 150 émergent de ladite surface ou sont localisées dans la masse au voisinage de ladite surface suivant une très faible épaisseur.

Suivant l'invention on préconise un procédé de préparation d'un filtre qui est caractérisé en ce que l'on soumet une préparation sous forme liquide ou pâteuse d'une substance polymère et d'un liquide de solvatation, dilution ou dispersion à (i) une congélation de - 40°C à - 80°C, (ii) un broyage du produit ainsi congelé, (iii) une cryodessiccation du broyat ainsi obtenu (i.e. sublimation du liquide de solvatation, dilution ou dispersion), puis (iv) une compression selon un taux de compression compris entre 1,1 et 5.

Suivant l'invention, on préconise une utilisation de l'élément filtrant 1 obtenu par congélation, broyage, cryodessiccation, et compression pour l'épuration et l'analyse de fluides gazeux. Cette utilisation consiste à faire passer le fluide à épurer ou à analyser à travers l'élément filtrant avec une perte de charge comprise entre 80 et 10 000 Pa.

Les exemples 4-5 qui suivent concernent plus particulièrement l'obtention de particules submicroniques de principes actifs insolubles dans l'eau.

## EXEMPLE 4

Une solution à 2,5 % (p/v) de nifédipine dans le chloroforme est nébulisée sous une pression de 4 bars, dans un courant porteur d'azote sec. On déssèche ensuite l'aérosol résultant à environ 30°C par circulation dans une boucle métallique dont la paroi est chauffée électriquement. L'aérosol de microparticules solides ainsi produit est entraîné vers un filtre à faible perte de charge et haute efficacité qui a été préparé comme indiqué à l'exemple 2 par compression ménagée d'un granulat lyophilisé obtenu à partir d'une composition aqueuse de Dextran 70 à 4 % (p/v), Pharmacoat 603 à 4 % (p/v) et saccharose à 0,0125 % (p/v). Dans les conditions optimales d'utilisation, l'élément filtrant retient l'ensemble des microparticules qui ont été produites, tandis que le fluide gazeux porteur, ici l'azote, qui a été enrichi des vapeurs de chloroforme évaporé et qui sort de l'élément filtrant, est dirigé vers une unité d'épuration, par exemple par condensation cryogénique permettant la séparation du solvant avant recyclage du fluide gazeux porteur.

En fin d'opération, l'ingrédient actif utilisé, dans le cas d'espèce la nifédipine, se trouve tout entier sous forme de particules submicroniques "piégées" dans la partie supérieure de l'élément filtrant fritté. On retire ensuite l'élément filtrant et on l'homogénéise pour disposer d'une matière première industrielle élaborée, riche en produit actif hautement divisé, qui peut être utilisée telle quelle, en tant que forme galénique, ou bien servir d'intermédiaire dans une succession d'autres opérations galéniques.

12

EP 0 298 864 B1

### EXEMPLE 5

On procède à la nébulisation de 700 g de solution chloroformique de nifédipine à 2,5 % (p/v) dans un gaz porteur, l'azote sec. On chauffe le gaz porteur à une température de 30-35°C pendant environ 50 à 70 minutes de façon que les microparticules liquides donnent des microparticules solides par évaporation du chloroforme.

On recueille les microparticules de nifédipine sur 60 g d'élément filtrant, sous forme de disque, préparé comme indiqué à l'exemple 4 ci-dessus.

Par analyse, le dosage de la nifédipine montre que l'on a

a) au voisinage de la surface dite d'entrée de l'élément filtrant : 2,4 % en poids de nifédipine ;

b) au voisinage du fond (i.e. la face de sortie) de l'élément filtrant : 0,09 % en poids de nifédipine ; et

c) après homogénéisation la teneur moyenne en nifédipine dans la matrice est de 0,58 % en poids.

Par ailleurs, la teneur en solvant résiduel est inférieure à 900 ppm (environ 800 - 900 ppm) avant homogénéisation, et inférieure à 400 ppm après homogénéisation.

Les photographies au microscope électronique à balayage montrent que les microparticules ont une granulométrie moyenne comprise entre 0,2 et 1$\mu$m.

Les exemples 6-9 qui suivent, illustrent l'obtention de particules submicroniques d'ingrédients actifs solubles dans l'eau, d'une part, et de particules submicroniques de principes actifs enrobés, d'autre part. Ils illustrent également l'obtention de la nouvelle forme galénique suivant l'invention.

### EXEMPLE 6

On prépare une solution aqueuse de chlorhydrate de Buflomédil à 5 % (p/v). On nébulise 100 g de cette solution sous une pression de 3-4 bars dans un courant d'azote sec. L'aérosol liquide résultant est séché pendant 50 minutes sous un gradient de température de 44°C (début du séchage) à 50°C (fin du séchage).

Les microparticules résultantes sont filtrées sur 45 g d'élément filtrant en polyvinylpyrrolidone obtenu par cryodessiccation puis compression.

Le dosage de l'élément filtrant permet de constater que la quantité de chlorhydrate de Buflomédil fixé sur l'élément filtrant est de 4 %.

Les photographies effectuées au microscope électronique à balayage montrent des points d'impact de l'ordre de 0,1$\mu$m à 0,2$\mu$m ce qui indique que l'on peut obtenir des particules ayant une granulométrie inférieure à 0,1$\mu$m.

### EXEMPLE 7

On procède comme indiqué à l'exemple 6 ci-dessus en nébulisant 100 g d'une solution aqueuse à 2 % (p/v) d'héparine, en séchant l'aérosol obtenu pendant 60 minutes avec un gradient de température de 60°C (début du séchage) à 50°C (fin du séchage) puis en recueillant les microparticules obtenues sur 40 g d'élément filtrant en polyvinylpyrrolidone cryodesséchée et comprimée.

Par dosage par photométrie de flamme, la quantité de sodium retrouvée sur l'élément filtrant correspond à une fixation exprimée en héparine sodique de l'ordre de 1 %. Les photographies obtenues par microscopie électronique à balayage montrent des particules régulières de l'ordre de 0,1 à 0,5$\mu$m, alors que les particules de départ étaient, lors de la dissolution dans l'eau, très hétérogènes de 10 à 100$\mu$m et plus.

### EXEMPLE 8

On procède comme indiqué à l'exemple 7 ci-dessus avec une solution aqueuse d'héparine à 5 % (p/v), c'est-à-dire une solution plus concentrée que celle de l'exemple 7.

L'examen au microscope électronique d'un prélèvement de la surface de l'élément filtrant confirme bien que les particules d'héparine, qui sont obtenues, sont de l'ordre de 0,1 à 0,5$\mu$m.

### EXEMPLE 9

On procède comme indiqué à l'exemple 7, en nébulisant 400 g d'une solution aqueuse d'héparine à 5 % (p/v) que l'on recueille sur un élément filtrant en polyvinylpyrrolidone (PVP) obtenu selon l'invention par congélation, broyage, cryodessiccation et compression.

13

On solubilise l'élément filtrant dans 500 ml de chloroforme, puis solubilise dans le mélange résultant une composition de glycérides et de polyglycérides partiels d'acides gras commercialisée sous le nom de marque de GELUCIRE. On nébulise la suspension résultante et recueille les microparticules enrobées sur un filtre en PVP conforme à l'invention. Lesdites microparticules d'héparine enrobées ainsi obtenues ont une granulométrie moyenne inférieure à 1$\mu$m ; elles peuvent être, le cas échéant, associées à des microparticules de GELUCIRE[R].

**Revendications**
**Revendications pour les Etats contractants suivants : AT, BE, CH, DE, FR, GB, IT, LI, LU, NL, SE**

1. Elément filtrant pour fluide gazeux, caractérisé en ce qu'il se présente sous la forme d'une couche poreuse et est constitué d'un matériau solide obtenu à l'état sec (i) par congélation, (ii) par cryodessiccation, puis (iii) par compression de façon à lui conférer une résistance mécanique suffisante pour éviter essentiellement qu'il ne se fissure lors du passage du fluide gazeux à travers son épaisseur.

2. Elément filtrant suivant la revendication 1, caractérisé en ce qu'il est constitué d'un matériau solide ayant été comprimé après cryodessiccation suivant un taux de compression compris entre 1,1 et 5.

3. Elément filtrant suivant la revendication 1, caractérisé en ce qu'il est constitué d'un matériau solide ayant été comprimé après cryodessiccation suivant un taux de compression compris entre 1,2 et 4.

4. Elément filtrant suivant la revendication 1, caractérisé en ce qu'il est constitué d'un matériau solide ayant été comprimé après cryodessiccation suivant un taux de compression compris entre 1,25 et 3,60.

5. Elément filtrant suivant la revendication 1, caractérisé en ce qu'il renferme dans sa masse un agent de cohésion choisi parmi l'ensemble constitué par les substances plastifiantes.

6. Elément filtrant suivant la revendication 5, caractérisé en ce que ledit agent de cohésion plastifiant est présent dans la masse du matériau solide de l'élément filtrant à une concentration comprise entre 1 % et 75 % en poids par rapport au poids total dudit matériau solide.

7. Elément filtrant suivant l'une quelconque des revendications 1 à 6, caractérisé en ce qu'il est constitué d'un matériau solide soluble ou dispersable dans un solvant choisi parmi l'ensemble comprenant l'eau, les solvants minéraux non aqueux, les solvants organiques et leurs mélanges.

8. Elément filtrant suivant l'une quelconque des revendications 1 à 7, caractérisé en ce qu'il est constitué d'un matériau solide obtenu à l'état sec (a) par congélation, (b) par broyage, (c) par cryodessiccation, puis (d) par compression.

9. Filtre pour fluide gazeux comprenant l'élément filtrant selon l'une quelconque des revendications 1 à 8.

10. Procédé de préparation de l'élément filtrant suivant la revendication 8, caractérisé en ce que l'on soumet une préparation sous forme liquide ou pâteuse d'une substance polymère et d'un liquide de solvatation, dilution ou dispersion à une congélation à une température de - 40°C à - 80°C, à un broyage du produit solide congelé ainsi obtenu, à une cryodessiccation du broyat résultant pour sublimer le liquide de solvatation, puis on soumet le produit cryodesséché ainsi obtenu à une compression à un taux compris entre 1,1 et 5.

11. Utilisation de l'élément filtrant suivant l'une quelconque des revendications 1 à 8, dans le domaine de l'épuration et de l'analyse d'impureté contenue dans un fluide gazeux, caractérisée en ce que l'on fait passer ledit fluide gazeux à travers ledit élément filtrant, disposé dans un filtre avec une perte de charge comprise entre 80 et 10 000 Pa.

12. Forme galénique comprenant en association (i) l'élément filtrant suivant l'une quelconque des revendications 1 à 8 en tant que matrice physiologiquement acceptable et (ii) au moins un ingrédient actif sous forme particulaire et choisi parmi les principes actifs thérapeutiques et cosmétiques, ladite forme galénique étant caractérisée en ce que ledit ingrédient actif associé à ladite matrice est constitué essentiellement par des microparticules ayant une granulométrie moyenne comprise entre 5$\mu$m et

14

0,01μm, et est choisi parmi les substances qui sont (1) solubles dans l'eau, (2) insolubles dans l'eau ou (3) enrobées.

13. Forme galénique suivant la revendication 12, caractérisée en ce que la matrice physiologiquement acceptable a été obtenue par congélation d'une solution aqueuse contenant 4 % (p/v) de dextrane, 4 % (p/v) d'hydroxypropylméthylcellulose et 0,0125 % (p/v) de saccharose, broyage, cryodessiccation puis compression du produit cryodesséché résultant à un taux compris entre 1,1 et 5.

14. Forme galénique suivant la revendication 12, caractérisée en ce que la matrice physiologiquement acceptable a été obtenue par congélation d'une solution contenant de la polyvinylpyrrolidone, broyage, cryodessiccation, puis compression du produit cryodesséché résultant à un taux compris entre 1,1 et 5.

15. Procédé de préparation de la forme galénique suivant la revendication 12, caractérisé en ce qu'il comprend les étapes suivantes :
1°) obtention d'une préparation de l'ingrédient actif dans un milieu liquide ledit ingrédient actif étant soit soluble, soit insoluble dans ledit milieu liquide et sa concentration dans ledit milieu liquide étant inférieure ou égale à 10 % (p/v) ;
2°) nébulisation de la préparation résultante pour obtenir un aérosol entraîné dans un courant de fluide gazeux porteur ;
3°) évaporation du milieu liquide de l'aérosol, de façon que les microparticules qui résultent du séchage dudit aérosol soient véhiculées par ledit fluide gazeux porteur ;
4°) filtration dudit fluide gazeux porteur contenant l'ingrédient actif essentiellement sous forme de microparticules au moyen d'un filtre comprenant, comme élément filtrant, un matériau solide et poreux qui a été obtenu à l'état sec par congélation, broyage, cryodessiccation et compression pour conférer audit matériau une résistance mécanique suffisante pour éviter essentiellement qu'il ne se fissure lors du passage d'un fluide gazeux à travers son épaisseur, pour recueillir sur l'élément filtrant lesdites microparticules.

16. Procédé suivant la revendication 15, dans lequel l'ingrédient actif est insoluble dans l'eau mais soluble dans un solvant organique, caractérisé en ce qu'il comprend les étapes suivantes :
1°) obtention d'une solution dans un solvant organique de l'ingrédient actif insoluble dans l'eau, de façon que la concentration dudit ingrédient actif dans ladite solution soit inférieure ou égale à 10 % (p/v) ;
2°) nébulisation de ladite solution ainsi obtenue sous forme d'aérosol liquide entraîné dans un courant de fluide gazeux porteur ;
3°) évaporation du solvant de l'ingrédient actif dans ledit courant de fluide gazeux porteur, de façon que les microparticules solides résultant de la dessiccation des microparticules liquides de l'aérosol soient véhiculées par ledit fluide gazeux porteur ;
4°) filtration dudit fluide gazeux porteur contenant l'ingrédient actif essentiellement sous forme de microparticules ayant une granulométrie moyenne comprise entre 5μm et 0,01μm, d'une part, et le solvant dudit ingrédient actif sous forme de vapeur, d'autre part, au moyen d'un filtre comprenant, comme élément filtrant, un matériau solide, poreux et hydrosoluble qui a été obtenu à l'état sec par congélation, broyage, cryodessiccation, et compression pour conférer audit matériau une résistance mécanique suffisante pour éviter essentiellement qu'il ne se fissure lors du passage d'un fluide gazeux à travers son épaisseur.

17. Procédé suivant la revendication 15, dans lequel l'ingrédient actif est soluble dans l'eau, caractérisé en ce qu'il comprend les étapes suivantes :
1°) obtention d'une solution dudit ingrédient actif dans l'eau, de façon que la concentration dudit ingrédient actif dans ladite solution soit inférieure ou égale à 10 % (p/v) ;
2°) nébulisation de ladite solution, ainsi obtenue, sous forme d'aérosol liquide entraîné dans un courant de fluide gazeux porteur ;
3°) évaporation de l'eau, solvant de l'ingrédient actif, dans ledit courant de fluide gazeux porteur, de façon que les microparticules solides qui résultent de la dessiccation des microparticules liquides de l'aérosol soient véhiculées par ledit fluide gazeux porteur ;
4°) filtration dudit fluide gazeux porteur contenant l'ingrédient actif essentiellement sous forme de microparticules ayant une granulométrie moyenne comprise entre 5μm et 0,01μm, d'une part, et le solvant dudit ingrédient actif sous forme de vapeur, d'autre part, au moyen d'un filtre comprenant, comme élément filtrant, un matériau solide et poreux qui a été obtenu à l'état sec par congélation, broyage, cryodessiccation et compression pour conférer audit matériau une résistance mécanique suffisante pour éviter essentiellement qu'il ne se fissure lors du passage d'un fluide gazeux à travers son épaisseur.

**18.** Procédé suivant la revendication 15, dans lequel l'ingrédient actif est sous la forme de microparticules enrobées, ledit procédé étant caractérisé en ce qu'il comprend les étapes suivantes :

1°) obtention d'une suspension de microparticules de l'ingrédient actif dans un milieu liquide contenant un matériau d'enrobage dissous, ledit ingrédient actif étant insoluble dans ledit milieu liquide et sa concentration dans ledit milieu liquide étant inférieure ou égale à 10 % (p/v) ;

2°) nébulisation de la suspension résultante pour obtenir un aérosol entraîné dans un courant de fluide gazeux porteur ;

3°) évaporation du milieu liquide de l'aérosol, de façon que les microparticules enrobées qui résultent du séchage dudit aérosol soient véhiculées par ledit fluide gazeux porteur ;

4°) filtration dudit fluide gazeux porteur contenant l'ingrédient actif essentiellement sous forme de microparticules enrobées par le matériau d'enrobage au moyen d'un filtre comprenant, comme élément filtrant, un matériau solide et poreux qui a été obtenu à l'état sec par congélation, broyage, cryodessiccation et compression pour conférer audit matériau une résistance mécanique suffisante pour éviter essentiellement qu'il ne se fissure lors du passage d'un fluide gazeux à travers son épaisseur, pour recueillir sur l'élément filtrant lesdites microparticules enrobées.

**19.** Procédé suivant l'une quelconque des revendications 16, 17 et 18, caractérisé en ce que l'on prépare à l'étape 1°) une préparation liquide ayant une teneur en ingrédient actif inférieure ou égale à 5 % (p/v).

**20.** Procédé suivant l'une quelconque des revendications 16, 17, et 18, caractérisé en ce que la nébulisation de l'étape 2°) est effectuée de façon à obtenir des particules d'aérosol ayant un diamètre moyen approprié pour donner, après évaporation du solvant de la préparation liquide, des microparticules solides ayant une granulométrie moyenne comprise entre $1\mu m$ et $0,01\mu m$.

**21.** Procédé suivant l'une quelconque des revendications 16, 17 et 18, caractérisé en ce que l'évaporation de l'étape 3°) est effectuée à une température comprise entre 25° et 90°C.

**22.** Procédé suivant l'une quelconque des revendications 16, 17 et 18, caractérisé en ce qu'il comprend en outre l'étape de malaxage de l'ensemble comprenant l'élément filtrant et les microparticules d'ingrédient actif et qui résulte de la filtration de l'étape 4°).

**23.** Procédé suivant la revendication 18, pour la préparation d'une forme galénique comprenant des microparticules d'ingrédient actif enrobées, caractérisé en ce qu'il comprend en outre, avant l'étape 1°), les étapes suivantes :

(a) obtention d'une solution de l'ingrédient actif dans un premier solvant, de façon que la concentration dudit ingrédient actif dans ledit premier solvant soit inférieure ou égale à 10 % (p/v) ;

(b) nébulisation de ladite solution, ainsi obtenue, sous forme d'aérosol liquide entraîné dans un courant de fluide gazeux porteur ;

(c) évaporation du solvant de l'ingrédient actif dans ledit courant de fluide gazeux porteur, de façon que les microparticules solides résultant de la dessiccation des microparticules liquides de l'aérosol soient véhiculées par ledit fluide gazeux porteur ;

(d) filtration dudit fluide gazeux porteur contenant l'ingrédient actif essentiellement sous forme de microparticules ayant une granulométrie moyenne comprise entre $5\mu m$ et $0,01\mu m$, d'une part, et le solvant dudit ingrédient actif sous forme de vapeur, d'autre part, au moyen d'un filtre comprenant, comme élément filtrant, un matériau solide et poreux qui a été obtenu à l'état sec par congélation, broyage, cryodessiccation et compression pour conférer audit matériau une résistance mécanique suffisante pour éviter essentiellement qu'il ne se fissure lors du passage d'un fluide gazeux à travers son épaisseur ;

(e) solubilisation de l'élément filtrant utilisé à l'étape (d) dans un second solvant comme décrit à l'étape 1°).

**24.** Utilisation du procédé suivant l'une quelconque des revendications 16, 17 et 18, caractérisée en ce que l'on recueille les microparticules de l'ingrédient actif, le cas échéant enrobées, par dissolution de la

EP 0 298 864 B1

matrice, qui lui est associée et qui a été utilisée comme élément filtrant au cours de la filtration de l'étape 4°), dans un solvant approprié ne dissolvant pas lesdites microparticules le cas échéant enrobées.

**Revendications pour les Etats contractants suivants : ES, GR**

1. Elément filtrant pour fluide gazeux, caractérisé en ce qu'il se présente sous la forme d'une couche poreuse et est constitué d'un matériau solide obtenu à l'état sec (i) par congélation, (ii) par cryodessiccation, puis (iii) par compression de façon à lui conférer une résistance mécanique suffisante pour éviter essentiellement qu'il ne se fissure lors du passage du fluide gazeux à travers son épaisseur.

2. Elément filtrant suivant la revendication 1, caractérisé en ce qu'il est constitué d'un matériau solide ayant été comprimé après cryodessiccation suivant un taux de compression compris entre 1,1 et 5.

3. Elément filtrant suivant la revendication 1, caractérisé en ce qu'il est constitué d'un matériau solide ayant été comprimé après cryodessiccation suivant un taux de compression compris entre 1,2 et 4.

4. Elément filtrant suivant la revendication 1, caractérisé en ce qu'il est constitué d'un matériau solide ayant été comprimé après cryodessiccation suivant un taux de compression compris entre 1,25 et 3,60.

5. Elément filtrant suivant la revendication 1, caractérisé en ce qu'il renferme dans sa masse un agent de cohésion choisi parmi l'ensemble constitué par les substances plastifiantes.

6. Elément filtrant suivant la revendication 5, caractérisé en ce que ledit agent de cohésion plastifiant est présent dans la masse du matériau solide de l'élément filtrant à une concentration comprise entre 1 % et 75 % en poids par rapport au poids total dudit matériau solide.

7. Elément filtrant suivant l'une quelconque des revendications 1 à 6, caractérisé en ce qu'il est constitué d'un matériau solide soluble ou dispersable dans un solvant choisi parmi l'ensemble comprenant l'eau, les solvants minéraux non aqueux, les solvants organiques et leurs mélanges.

8. Elément filtrant suivant l'une quelconque des revendications 1 à 7, caractérisé en ce qu'il est constitué d'un matériau solide obtenu à l'état sec (a) par congélation, (b) par broyage, (c) par cryodessiccation, puis (d) par compression.

9. Filtre pour fluide gazeux comprenant l'élément filtrant selon l'une quelconque des revendications 1 à 8.

10. Procédé de préparation de l'élément filtrant suivant la revendication 8, caractérisé en ce que l'on soumet une préparation sous forme liquide ou pâteuse d'une substance polymère et d'un liquide de solvatation, dilution ou dispersion à une congélation à une température de - 40°C à - 80°C, à un broyage du produit solide congelé ainsi obtenu, à une cryodessiccation du broyat résultant pour sublimer le liquide de solvatation, puis on soumet le produit cryodesséché ainsi obtenu à une compression à un taux compris entre 1,1 et 5.

11. Utilisation de l'élément filtrant suivant l'une quelconque des revendications 1 à 8, dans le domaine de l'épuration et de l'analyse d'impureté contenue dans un fluide gazeux, caractérisée en ce que l'on fait passer ledit fluide gazeux à travers ledit élément filtrant, disposé dans un filtre avec une perte de charge comprise entre 80 et 10 000 Pa.

12. Procédé de préparation d'une forme galénique comprenant en association (i) l'élément filtrant suivant l'une quelconque des revendications 1 à 8 en tant que matrice physiologiquement acceptable et (ii) au moins un ingrédient actif sous forme particulaire et choisi parmi les principes actifs thérapeutiques et cosmétiques, ledit ingrédient actif associé à ladite matrice étant constitué essentiellement par des microparticules ayant une granulométrie moyenne comprise entre 5μm et 0,01μm, et étant choisi parmi les substances qui sont (1) solubles dans l'eau, (2) insolubles dans l'eau ou (3) enrobées, ledit procédé étant caractérisé en ce qu'il comprend les étapes suivantes :
  1°) obtention d'une préparation de l'ingrédient actif dans un milieu liquide ledit ingrédient actif étant soit soluble, soit insoluble dans ledit milieu liquide et sa concentration dans ledit milieu liquide étant

17

EP 0 298 864 B1

inférieure ou égale à 10 % (p/v) ;

2°) nébulisation de la préparation résultante pour obtenir un aérosol entraîné dans un courant de fluide gazeux porteur ;

3°) évaporation du milieu liquide de l'aérosol, de façon que les microparticules qui résultent du séchage dudit aérosol soient véhiculées par ledit fluide gazeux porteur ;

4°) filtration dudit fluide gazeux porteur contenant l'ingrédient actif essentiellement sous forme de microparticules au moyen d'un filtre comprenant, comme élément filtrant, un matériau solide et poreux qui a été obtenu à l'état sec par congélation, broyage, cryodessiccation et compression pour conférer audit matériau une résistance mécanique suffisante pour éviter essentiellement qu'il ne se fissure lors du passage d'un fluide gazeux à travers son épaisseur, pour recueillir sur l'élément filtrant lesdites microparticules.

13. Procédé suivant la revendication 12, dans lequel l'ingrédient actif est insoluble dans l'eau mais soluble dans un solvant organique, caractérisé en ce qu'il comprend les étapes suivantes :

1°) obtention d'une solution dans un solvant organique de l'ingrédient actif insoluble dans l'eau, de façon que la concentration dudit ingrédient actif dans ladite solution soit inférieure ou égale à 10 % (p/v) ;

2°) nébulisation de ladite solution ainsi obtenue sous forme d'aérosol liquide entraîné dans un courant de fluide gazeux porteur ;

3°) évaporation du solvant de l'ingrédient actif dans ledit courant de fluide gazeux porteur, de façon que les microparticules solides résultant de la dessiccation des microparticules liquides de l'aérosol soient véhiculées par ledit fluide gazeux porteur ;

4°) filtration dudit fluide gazeux porteur contenant l'ingrédient actif essentiellement sous forme de microparticules ayant une granulométrie moyenne comprise entre 5$\mu$m et 0,01$\mu$m, d'une part, et le solvant dudit ingrédient actif sous forme de vapeur, d'autre part, au moyen d'un filtre comprenant, comme élément filtrant, un matériau solide, poreux et hydrosoluble qui a été obtenu à l'état sec par congélation, broyage, cryodessiccation, et compression pour conférer audit matériau une résistance mécanique suffisante pour éviter essentiellement qu'il ne se fissure lors du passage d'un fluide gazeux à travers son épaisseur.

14. Procédé suivant la revendication 12, dans lequel l'ingrédient actif est soluble dans l'eau, caractérisé en ce qu'il comprend les étapes suivantes :

1°) obtention d'une solution dudit ingrédient actif dans l'eau, de façon que la concentration dudit ingrédient actif dans ladite solution soit inférieure ou égale à 10 % (p/v) ;

2°) nébulisation de ladite solution, ainsi obtenue, sous forme d'aérosol liquide entraîné dans un courant de fluide gazeux porteur ;

3°) évaporation de l'eau, solvant de l'ingrédient actif, dans ledit courant de fluide gazeux porteur, de façon que les microparticules solides qui résultent de la dessiccation des microparticules liquides de l'aérosol soient véhiculées par ledit fluide gazeux porteur ;

4°) filtration dudit fluide gazeux porteur contenant l'ingrédient actif essentiellement sous forme de microparticules ayant une granulométrie moyenne comprise entre 5$\mu$m et 0,01$\mu$m, d'une part, et le solvant dudit ingrédient actif sous forme de vapeur, d'autre part, au moyen d'un filtre comprenant, comme élément filtrant, un matériau solide et poreux qui a été obtenu à l'état sec par congélation, broyage, cryodessiccation et compression pour conférer audit matériau une résistance mécanique suffisante pour éviter essentiellement qu'il ne se fissure lors du passage d'un fluide gazeux à travers son épaisseur.

15. Procédé suivant la revendication 12, dans lequel l'ingrédient actif est sous la forme de microparticules enrobées, ledit procédé étant caractérisé en ce qu'il comprend les étapes suivantes :

1°) obtention d'une suspension de microparticules de l'ingrédient actif dans un milieu liquide contenant un matériau d'enrobage dissous, ledit ingrédient actif étant insoluble dans ledit milieu liquide et sa concentration dans ledit milieu liquide étant inférieure ou égale à 10 % (p/v) ;

2°) nébulisation de la suspension résultante pour obtenir un aérosol entraîné dans un courant de fluide gazeux porteur ;

3°) évaporation du milieu liquide de l'aérosol, de façon que les microparticules enrobées qui résultent du séchage dudit aérosol soient véhiculées par ledit fluide gazeux porteur ;

4°) filtration dudit fluide gazeux porteur contenant l'ingrédient actif essentiellement sous forme de microparticules enrobées par le matériau d'enrobage au moyen d'un filtre comprenant, comme

18

élément filtrant, un matériau solide et poreux qui a été obtenu à l'état sec par congélation, broyage, cryodessiccation et compression pour conférer audit matériau une résistance mécanique suffisante pour éviter essentiellement qu'il ne se fissure lors du passage d'un fluide gazeux à travers son épaisseur, pour recueillir sur l'élément filtrant lesdites microparticules enrobées.

16. Procédé suivant l'une quelconque des revendications 13, 14 et 15, caractérisé en ce que l'on prépare à l'étape 1°) une préparation liquide ayant une teneur en ingrédient actif inférieure ou égale à 5 % (p/v).

17. Procédé suivant l'une quelconque des revendications 13, 14 et 15, caractérisé en ce que la nébulisation de l'étape 2°) est effectuée de façon à obtenir des particules d'aérosol ayant un diamètre moyen approprié pour donner, après évaporation du solvant de la préparation liquide, des microparticules solides ayant une granulométrie moyenne comprise entre 1$\mu$m et 0,01$\mu$m.

18. Procédé suivant l'une quelconque des revendications 13, 14 et 15, caractérisé en ce que l'évaporation de l'étape 3°) est effectuée à une température comprise entre 25° et 90°C.

19. Procédé suivant l'une quelconque des revendications 13, 14 et 15, caractérisé en ce qu'il comprend en outre l'étape de malaxage de l'ensemble comprenant l'élément filtrant et les microparticules d'ingrédient actif et qui résulte de la filtration de l'étape 4°).

20. Procédé suivant la revendication 15, pour la préparation d'une forme galénique comprenant des microparticules d'ingrédient actif enrobées, caractérisé en ce qu'il comprend en outre, avant l'étape 1°), les étapes suivantes :
    (a) obtention d'une solution de l'ingrédient actif dans un premier solvant, de façon que la concentration dudit ingrédient actif dans ledit premier solvant soit inférieure ou égale à 10 % (p/v) ;
    (b) nébulisation de ladite solution, ainsi obtenue, sous forme d'aérosol liquide entraîné dans un courant de fluide gazeux porteur ;
    (c) évaporation du solvant de l'ingrédient actif dans ledit courant de fluide gazeux porteur, de façon que les microparticules solides résultant de la dessiccation des microparticules liquides de l'aérosol soient véhiculées par ledit fluide gazeux porteur ;
    (d) filtration dudit fluide gazeux porteur contenant l'ingrédient actif essentiellement sous forme de microparticules ayant une granulométrie moyenne comprise entre 5$\mu$m et 0,01$\mu$m, d'une part, et le solvant dudit ingrédient actif sous forme de vapeur, d'autre part, au moyen d'un filtre comprenant, comme élément filtrant, un matériau solide et poreux qui a été obtenu à l'état sec par congélation, broyage, cryodessiccation et compression pour conférer audit matériau une résistance mécanique suffisante pour éviter essentiellement qu'il ne se fissure lors du passage d'un fluide gazeux à travers son épaisseur ;
    (e) solubilisation de l'élément filtrant utilisé à l'étape (d) dans un second solvant comme décrit à l'étape 1°).

21. Utilisation du procédé suivant l'une quelconque des revendications 13, 14 et 15, caractérisée en ce que l'on recueille les microparticules de l'ingrédient actif, le cas échéant enrobées, par dissolution de la matrice, qui lui est associée et qui a été utilisée comme élément filtrant au cours de la filtration de l'étape 4°), dans un solvant approprié ne dissolvant pas lesdites microparticules le cas échéant enrobées.

**Claims**
**Claims for the following Contracting States : AT, BE, CH, DE, FR, GB, IT, LI, LU, NL, SE**

1. A filter element for gaseous fluid, characterized in that it is in the form of a porous layer and consists of a solid material obtained in the dry state by (i) freezing, (ii) freeze-drying and then (iii) compression in order to give it sufficient mechanical strength essentially to prevent it from cracking when the gaseous fluid passes through it.

2. A filter element according to Claim 1, characterized in that it consists of a solid material which has been compressed, after freeze-drying, in a compression ratio of between 1.1 and 5.

3. A filter element according to Claim 1, characterized in that it consists of a solid material which has been

EP 0 298 864 B1

compressed, after freeze-drying, in a compression ratio of between 1.2 and 4.

4. A filter element according to Claim 1, characterized in that it consists of a solid material which has been compressed, after freeze-drying, in a compression ratio of between 1.25 and 3.60.

5. A filter element according to Claim 1, characterized in that it contains, in its bulk, a cohesive agent selected from the group comprising plasticizing substances.

6. A filter element according to Claim 5, characterized in that said plasticizing cohesive agent is present in the bulk of the solid material of the filter element at a concentration of between 1% and 75% by weight, relative to the total weight of said solid material.

7. A filter element according to any one of Claims 1 to 6, characterized in that it consists of a solid material which is soluble or dispersible in a solvent selected from the group comprising water, non-aqueous inorganic solvents, organic solvents and mixtures thereof.

8. A filter element according to any one of Claims 1 to 7, characterized in that it consists of a solid material obtained in the dry state by (a) freezing, (b) grinding, (c) freeze-drying and then (d) compression.

9. A filter for gaseous fluid, comprising the filter element according to any one of Claims 1 to 8.

10. A method of preparing the filter element according to Claim 8, characterized in that a liquid or pasty preparation of a polymeric substance and a solvating, diluting or dispersing liquid is frozen at a temperature of -40°C to -80°C, the resulting frozen solid product is ground and the resulting ground product is freeze-dried in order to sublime the solvating liquid, and then the resulting freeze-dried product is compressed in a ratio of between 1.1 and 5.

11. Use of the filter element according to any one of Claims 1 to 8 in the field of the purification and analysis of impurities contained in a gaseous fluid, characterized in that said gaseous fluid is passed through said filter element, arranged in a filter, with a pressure loss of between 80 and 10,000 Pa.

12. A pharmaceutical form comprising an association of (i) the filter element according to any one of Claims 1 to 8 as a physiologically acceptable matrix, and (ii) at least one active ingredient in the form of particles, which is selected from therapeutic and cosmetic active principles, said pharmaceutical form being characterized in that said active ingredient associated with said matrix consists essentially of microparticles with a mean size of between 5 $\mu$m and 0.01 $\mu$m and is selected from substances which are (1) soluble in water, (2) insoluble in water or (3) coated.

13. A pharmaceutical form according to Claim 12, characterized in that the physiologically acceptable matrix has been obtained by freezing of an aqueous solution containing 4% (w/v) of dextran, 4% (w/v) of hydroxypropyl methyl cellulose and 0.0125% (w/v) of sucrose, grinding, freeze-drying and then compression of the resulting freeze-dried product in a ratio of between 1.1 and 5.

14. A pharmaceutical form according to Claim 12, characterized in that the physiologically acceptable matrix has been obtained by freezing of a solution containing polyvinylpyrrolidone, grinding, freeze-drying and then compression of the resulting freeze-dried product in a ratio of between 1.1 and 5.

15. A method of preparing the pharmaceutical form according to Claim 12, characterized in that it comprises the following steps:
1°) production of a preparation of the active ingredient in a liquid medium, said active ingredient being either soluble or insoluble in said liquid medium and its concentration in said liquid medium being less than or equal to 10% (w/v);
2°) nebulization of the resulting preparation to give an aerosol entrained in a stream of gaseous carrier fluid;
3°) evaporation of the liquid medium of the aerosol so that the microparticles which result from the drying of said aerosol are conveyed by said gaseous carrier fluid; and
4°) filtration of said gaseous carrier fluid, containing the active ingredient essentially in the form of

20

microparticles, by means of a filter comprising, as the filter element, a porous solid material which has been obtained in the dry state by freezing, grinding, freeze-drying and compression in order to give said material sufficient mechanical strength essentially to prevent it from cracking when a gaseous fluid passes through it for collection of said microparticles on the filter element.

16. A method according to Claim 15 in which the active ingredient is insoluble in water but soluble in an organic solvent, characterized in that it comprises the following steps:

1°) preparation of a solution of the water-insoluble active ingredient in an organic solvent so that the concentration of said active ingredient in said solution is less than or equal to 10% (w/v);

2°) nebulization of said solution, obtained in this way, in the form of a liquid aerosol entrained in a stream of gaseous carrier fluid;

3°) evaporation of the solvent for the active ingredient in said stream of gaseous carrier fluid so that the solid microparticles resulting from the drying of the liquid microparticles of the aerosol are conveyed by said gaseous carrier fluid; and

4°) filtration of said gaseous carrier fluid, containing the active ingredient essentially in the form of microparticles with a mean size of between 5 $\mu$m and 0.01 $\mu$m, on the one hand, and the solvent for said active ingredient in the form of vapour, on the other, by means of a filter comprising, as the filter element, a solid, porous and water-soluble material which has been obtained in the dry state by freezing, grinding, freeze-drying and compression in order to give said material sufficient mechanical strength essentially to prevent it from cracking when a gaseous fluid passes through it.

17. A method according to Claim 15 in which the active ingredient is soluble in water, characterized in that it comprises the following steps:

1°) preparation of a solution of said active ingredient in water so that the concentration of said active ingredient in said solution is less than or equal to 10% (w/v);

2°) nebulization of said solution, obtained in this way, in the form of a liquid aerosol entrained in a stream of gaseous carrier fluid;

3°) evaporation of the water, which is the solvent for the active ingredient, in said stream of gaseous carrier fluid so that the solid microparticles which result from the drying of the liquid microparticles of the aerosol are conveyed by said gaseous carrier fluid; and

4°) filtration of said gaseous carrier fluid, containing the active ingredient essentially in the form of microparticles with a mean size of between 5 $\mu$m and 0.01 $\mu$m, on the one hand, and the solvent for said active ingredient in the form of vapour, on the other, by means of a filter comprising, as the filter element, a porous solid material which has been obtained in the dry state by freezing, grinding, freeze-drying and compression in order to give said material sufficient mechanical strength essentially to prevent it from cracking when a gaseous fluid passes through it.

18. A method according to Claim 15 in which the active ingredient is in the form of coated microparticles, said method being characterized in that it comprises the following steps:

1°) preparation of a suspension of microparticles of the active ingredient in a liquid medium containing a dissolved coating material, said active ingredient being insoluble in said liquid medium and its concentration in said liquid medium being less than or equal to 10% (w/v);

2°) nebulization of the resulting suspension to give an aerosol entrained in a stream of gaseous carrier fluid;

3°) evaporation of the liquid medium of the aerosol so that the coated microparticles which result from the drying of said aerosol are conveyed by said gaseous carrier fluid; and

4°) filtration of said gaseous carrier fluid, containing the active ingredient essentially in the form of microparticles coated with the coating material, by means of a filter comprising, as the filter element, a porous solid material which has been obtained in the dry state by freezing, grinding, freeze-drying and compression in order to give said material sufficient mechanical strength essentially to prevent it from cracking when a gaseous fluid passes through it for collection of said coated microparticles on the filter element.

19. A method according to any one of Claims 16, 17 and 18, characterized in that a liquid preparation in which the proportion of active ingredient is less than or equal to 5% (w/v) is prepared in step 1°).

20. A method according to any one of Claims 16, 17 and 18, characterized in that the purpose of the nebulization of step 2°) is to obtain aerosol particles whose mean diameter is such that, after evaporation of the solvent of the liquid preparation, solid microparticles are obtained which have a mean size of between 1 μm and 0.01 μm.

21. A method according to any one of Claims 16, 17 and 18, characterized in that the evaporation of step 3°) is carried out at a temperature of between 25° and 90°C.

22. A method according to any one of Claims 16, 17 and 18, characterized in that it also comprises a step for malaxation of the whole made up of the filter element and the microparticles of active ingredient, which results from the filtration of step 4°).

23. A method according to Claim 18 for the preparation of a pharmaceutical form comprising coated microparticles of active ingredient, characterized in that it also comprises, before step 1°), the following steps:
    (a) preparation of a solution of the active ingredient in a first solvent so that the concentration of said active ingredient in said first solvent is less than or equal to 10% (w/v);
    (b) nebulization of said solution, obtained in this way, in the form of a liquid aerosol entrained in a stream of gaseous carrier fluid;
    (c) evaporation of the solvent for the active ingredient in said stream of gaseous carrier fluid so that the solid microparticles resulting from the drying of the liquid microparticles of the aerosol are conveyed by said gaseous carrier fluid;
    (d) filtration of said gaseous carrier fluid, containing the active ingredient essentially in the form of microparticles with a mean size of between 5 μm and 0.01 μm, on the one hand, and the solvent for said active ingredient in the form of vapour, on the other, by means of a filter comprising, as the filter element, a porous solid material which has been obtained in the dry state by freezing, grinding, freeze-drying and compression in order to give said material sufficient mechanical strength essentially to prevent it from cracking when a gaseous fluid passes through it; and
    (e) solubilization of the filter element used in step (d) in a second solvent, as described in step 1°).

24. Use of the method according to any one of Claims 16, 17 and 18, characterized in that the microparticles of the active ingredient, which may or may not be coated, are collected by dissolution of the matrix, which is associated therewith and which has been used as the filter element during the filtration of step 4°), in an appropriate solvent in which said coated or uncoated microparticles are insoluble.

**Claims for the following Contracting States : ES, GR**

1. A filter element for gaseous fluid, characterized in that it is in the form of a porous layer and consists of a solid material obtained in the dry state by (i) freezing, (ii) freeze-drying and then (iii) compression in order to give it sufficient mechanical strength essentially to prevent it from cracking when the gaseous fluid passes through it.

2. A filter element according to Claim 1, characterized in that it consists of a solid material which has been compressed, after freeze-drying, in a compression ratio of between 1.1 and 5.

3. A filter element according to Claim 1, characterized in that it consists of a solid material which has been compressed, after freeze-drying, in a compression ratio of between 1.2 and 4.

4. A filter element according to Claim 1, characterized in that it consists of a solid material which has been compressed, after freeze-drying, in a compression ratio of between 1.25 and 3.60.

5. A filter element according to Claim 1, characterized in that it contains, in its bulk, a cohesive agent selected from the group comprising plasticizing substances.

6. A filter element according to Claim 5, characterized in that said plasticizing cohesive agent is present in the bulk of the solid material of the filter element at a concentration of between 1% and 75% by weight, relative to the total weight of said solid material.

7. A filter element according to any one of Claims 1 to 6, characterized in that it consists of a solid

22

material which is soluble or dispersible in a solvent selected from the group comprising water, non-aqueous inorganic solvents, organic solvents and mixtures thereof.

8. A filter element according to any one of Claims 1 to 7, characterized in that it consists of a solid material obtained in the dry state by (a) freezing, (b) grinding, (c) freeze-drying and then (d) compression.

9. A filter for gaseous fluid, comprising the filter element according to any one of Claims 1 to 8.

10. A method of preparing the filter element according to Claim 8, characterized in that a liquid or pasty preparation of a polymeric substance and a solvating, diluting or dispersing liquid is frozen at a temperature of -40°C to -80°C, the resulting frozen solid product is ground and the resulting ground product is freeze-dried in order to sublime the solvating liquid, and then the resulting freeze-dried product is compressed in a ratio of between 1.1 and 5.

11. Use of the filter element according to any one of Claims 1 to 8 in the field of the purification and analysis of impurities contained in a gaseous fluid, characterized in that said gaseous fluid is passed through said filter element, arranged in a filter, with a pressure loss of between 80 and 10,000 Pa.

12. A method of preparing a pharmaceutical form comprising an association of (i) the filter element according to any one of Claims 1 to 8 as a physiologically acceptable matrix, and (ii) at least one active ingredient in the form of particles, which is selected from therapeutic and cosmetic active principles, said active ingredient associated with said matrix consisting essentially of microparticles with a mean size of between 5 $\mu$m and 0.01 $\mu$m and being selected from substances which are (1) soluble in water, (2) insoluble in water or (3) coated, said method being characterized in that it comprises the following steps:

1°) production of a preparation of the active ingredient in a liquid medium, said active ingredient being either soluble or insoluble in said liquid medium and its concentration in said liquid medium being less than or equal to 10% (w/v);

2°) nebulization of the resulting preparation to give an aerosol entrained in a stream of gaseous carrier fluid;

3°) evaporation of the liquid medium of the aerosol so that the microparticles which result from the drying of said aerosol are conveyed by said gaseous carrier fluid; and

4°) filtration of said gaseous carrier fluid, containing the active ingredient essentially in the form of microparticles, by means of a filter comprising, as the filter element, a porous solid material which has been obtained in the dry state by freezing, grinding, freeze-drying and compression in order to give said material sufficient mechanical strength essentially to prevent it from cracking when a gaseous fluid passes through it for collection of said microparticles on the filter element.

13. A method according to Claim 12 in which the active ingredient is insoluble in water but soluble in an organic solvent, characterized in that it comprises the following steps:

1°) preparation of a solution of the water-insoluble active ingredient in an organic solvent so that the concentration of said active ingredient in said solution is less than or equal to 10% (w/v);

2°) nebulization of said solution, obtained in this way, in the form of a liquid aerosol entrained in a stream of gaseous carrier fluid;

3°) evaporation of the solvent for the active ingredient in said stream of gaseous carrier fluid so that the solid microparticles resulting from the drying of the liquid microparticles of the aerosol are conveyed by said gaseous carrier fluid; and

4°) filtration of said gaseous carrier fluid, containing the active ingredient essentially in the form of microparticles with a mean size of between 5 $\mu$m and 0.01 $\mu$m, on the one hand, and the solvent for said active ingredient in the form of vapour, on the other, by means of a filter comprising, as the filter element, a solid, porous and water-soluble material which has been obtained in the dry state by freezing, grinding, freeze-drying and compression in order to give said material sufficient mechanical strength essentially to prevent it from cracking when a gaseous fluid passes through it.

14. A method according to Claim 12 in which the active ingredient is soluble in water, characterized in that it comprises the following steps:

1°) preparation of a solution of said active ingredient in water so that the concentration of said active

23

ingredient in said solution is less than or equal to 10% (w/v);

2°) nebulization of said solution, obtained in this way, in the form of a liquid aerosol entrained in a stream of gaseous carrier fluid;

3°) evaporation of the water, which is the solvent for the active ingredient, in said stream of gaseous carrier fluid so that the solid microparticles which result from the drying of the liquid microparticles of the aerosol are conveyed by said gaseous carrier fluid; and

4°) filtration of said gaseous carrier fluid, containing the active ingredient essentially in the form of microparticles with a mean size of between 5 $\mu$m and 0.01 $\mu$m, on the one hand, and the solvent for said active ingredient in the form of vapour, on the other, by means of a filter comprising, as the filter element, a porous solid material which has been obtained in the dry state by freezing, grinding, freeze-drying and compression in order to give said material sufficient mechanical strength essentially to prevent it from cracking when a gaseous fluid passes through it.

**15.** A method according to Claim 12 in which the active ingredient is in the form of coated microparticles, said method being characterized in that it comprises the following steps:

1°) preparation of a suspension of microparticles of the active ingredient in a liquid medium containing a dissolved coating material, said active ingredient being insoluble in said liquid medium and its concentration in said liquid medium being less than or equal to 10% (w/v);

2°) nebulization of the resulting suspension to give an aerosol entrained in a stream of gaseous carrier fluid;

3°) evaporation of the liquid medium of the aerosol so that the coated microparticles which result from the drying of said aerosol are conveyed by said gaseous carrier fluid; and

4°) filtration of said gaseous carrier fluid, containing the active ingredient essentially in the form of microparticles coated with the coating material, by means of a filter comprising, as the filter element, a porous solid material which has been obtained in the dry state by freezing, grinding, freeze-drying and compression in order to give said material sufficient mechanical strength essentially to prevent it from cracking when a gaseous fluid passes through it for collection of said coated microparticles on the filter element.

**16.** A method according to any one of Claims 13, 14 and 15, characterized in that a liquid preparation in which the proportion of active ingredient is less than or equal to 5% (w/v) is prepared in step 1°).

**17.** A method according to any one of Claims 13, 14 and 15, characterized in that the purpose of the nebulization of step 2°) is to obtain aerosol particles whose mean diameter is such that, after evaporation of the solvent of the liquid preparation, solid microparticles are obtained which have a mean size of between 1 $\mu$m and 0.01 $\mu$m.

**18.** A method according to any one of Claims 13, 14 and 15, characterized in that the evaporation of step 3°) is carried out at a temperature of between 25° and 90°C.

**19.** A method according to any one of Claims 13, 14 and 15, characterized in that it also comprises a step for malaxation of the whole made up of the filter element and the microparticles of active ingredient, which results from the filtration of step 4°).

**20.** A method according to Claim 15 for the preparation of a pharmaceutical form comprising coated microparticles of active ingredient, characterized in that it also comprises, before step 1°), the following steps:

(a) preparation of a solution of the active ingredient in a first solvent so that the concentration of said active ingredient in said first solvent is less than or equal to 10% (w/v);

(b) nebulization of said solution, obtained in this way, in the form of a liquid aerosol entrained in a stream of gaseous carrier fluid;

(c) evaporation of the solvent for the active ingredient in said stream of gaseous carrier fluid so that the solid microparticles resulting from the drying of the liquid microparticles of the aerosol are conveyed by said gaseous carrier fluid;

(d) filtration of said gaseous carrier fluid, containing the active ingredient essentially in the form of microparticles with a mean size of between 5 $\mu$m and 0.01 $\mu$m, on the one hand, and the solvent for said active ingredient in the form of vapour, on the other, by means of a filter comprising, as the filter element, a porous solid material which has been obtained in the dry state by freezing, grinding, freeze-drying and compression in order to give said material sufficient mechanical strength essentially to prevent it from cracking when a gaseous fluid passes through it; and
(e) solubilization of the filter element used in step (d) in a second solvent, as described in step 1°).

21. Use of the method according to any one of Claims 13, 14 and 15, characterized in that the microparticles of the active ingredient, which may or may not be coated, are collected by dissolution of the matrix, which is associated therewith and which has been used as the filter element during the filtration of step 4°), in an appropriate solvent in which said coated or uncoated microparticles are insoluble.

**Patentansprüche**
**Patentansprüche für folgende Vertragsstaaten : AT, BE, CH, DE, FR, GB, IT, LI, LU, NL, SE**

1. Filterelement für ein gasförmiges Fluid,
dadurch **gekennzeichnet,**
daß es in der Form einer porösen Schicht vorliegt und gebildet ist aus einem festen Material, das in dem trockenen Zustand erhalten wurde, durch (i) Gefrieren, (ii) Gefriertrocknung und dann (iii) Verdichtung zwecks Verleihung einer hinreichenden mechanischen Festigkeit zur weitgehenden Vermeidung eines Spaltens während des Durchtritts des gasförmigen Fluids durch die Dickenerstreckung hindurch.

2. Filterelement gemäß Anspruch 1,
dadurch **gekennzeichnet,**
daß es gebildet ist aus einem festen Material, das nach der Gefriertrocknung entsprechend einem Kompressionsverhältnis zwischen 1,1 und 5 verdichtet wurde.

3. Filterelement gemäß Anspruch 1,
dadurch **gekennzeichnet,**
daß es gebildet ist aus einem festen Material, das nach der Gefriertrocknung entsprechend einem Kompressionsverhältnis zwischen 1,2 und 4 verdichtet wurde.

4. Filterelement gemäß Anspruch 1,
dadurch **gekennzeichnet,**
daß es gebildet ist aus einem festen Material, das nach der Gefriertrocknung entsprechend einem Kompressionsverhältnis zwischen 1,25 und 3,60 verdichtet wurde.

5. Filterelement gemäß Anspruch 1,
dadurch **gekennzeichnet,**
daß es in seiner Masse ein Kohäsionsmittel enthält, das ausgewählt ist aus der Gruppe der plastifizierenden Substanzen.

6. Filterelement gemäß Anspruch 5,
dadurch **gekennzeichnet,**
daß das plastifizierende Kohäsionsmittel in der Masse des festen Materials vom Filterelement in einer Konzentration zwischenll und 75 Gew.-%, bezogen auf das Gesamtgewicht des festen Materials, vorliegt.

7. Filterelement gemäß irgendeinem der Ansprüche 1 bis 6,
dadurch **gekennzeichnet,**
daß es aus einem festen Material gebildet ist, das löslich oder dispergierbar in einem Lösungsmittel aus der Gruppe Wasser, nichtwässerige mineralische Lösungsmittel, organische Lösungsmittel und deren Mischungen ist.

8. Filterelement gemäß irgendeinem der Ansprüche 1 bis 7,
dadurch **gekennzeichnet,**
daß es gebildet ist aus einem festen Material, das in dem trockenen Zustand erhalten wird durch (a) Gefrieren, (b) Zerkleinern, (c) Gefriertrocknung und dann (d) Verdichtung.

**9.** Filter für ein gasförmiges Fluid, enthaltend ein Filterelement gemäß einem der Ansprüche 1 bis 8.

**10.** Verfahren zur Herstellung des Filterelements gemäß Anspruch 8,
dadurch **gekennzeichnet,**
daß man einen in flüssiger oder pastöser Form vorliegenden Ansatz aus einer polymeren Substanz und einem lösenden, verdünnenden oder dispergierenden Medium einer Temperatur von -40 °C bis -80 °C unterwirft, das erhaltene verfestigte und gefrorene Produkt zerkleinert, dann eine Gefriertrocknung des Mahlguts zum Sublimieren der Verteilungsflüssigkeit durchführt und das erhaltene gefriergetrocknete Produkt bei einem Verdichtungsverhältnis zwischen 1,1 und 5 verdichtet.

**11.** Verwendung eines Filterelements gemäß irgendeinem der Ansprüche 1 bis 8 auf dem Gebiet der Reinigung und Analyse von in einem gasförmigen Fluid enthaltenen Beimengungen,
dadurch **gekennzeichnet,**
daß man das gasförmige Fluid durch das in einem Filter angeordnete Filterelement bei einem Spannungsverlust zwischen 80 bis 10 000 Pa zum Durchströmen bringt.

**12.** Galenische Form, enthaltend in Assoziation (i) ein Filterelement gemäß irgendeinem der Ansprüche 1 bis 8 als physiologisch akzeptable Matrix und (ii) wenigstens einen teilchenförmigen Wirkstoff, ausgewählt aus therapeutischen und kosmetischen Bestandteilen,
dadurch **gekennzeichnet,**
daß der als physiologisch akzeptable, an die Matrix gebundene Wirkstoff im wesentlichen von Mikroteilchen mit einer mittleren Kornverteilung zwischen 5 $\mu$m und 0,01 $\mu$m gebildet wird und ausgewählt ist aus Substanzen, die (1) löslich in Wasser, (2) unlöslich in Wasser oder (3) umhüllt sind.

**13.** Galenische Form gemäß Anspruch 12,
dadurch **gekennzeichnet,**
daß die physiologisch akzeptable Matrix erhalten worden ist durch Gefrieren einer wässerigen Lösung, die 4% (G/V) an Dextran, 4% (G/V) an Hydroxypropylmethylcellulose und 0,0125% (G/V) an Saccharose enthält, Zerkleinern, Gefriertrocknen und anschließendes Verdichten des gefriergetrockneten Produkts bei einem Verdichtungsverhältnis zwischen 1,1 und 5.

**14.** Galenische Form gemäß Anspruch 12,
dadurch **gekennzeichnet,**
daß die physiologisch akzeptable Matrix erhalten worden ist durch Gefrieren einer wässerigen Lösung mit einem Gehalt an Polyvinylpyrrolidon, Zerkleinern, Gefriertrocknen und nachfolgendes Verdichten des gefriergetrockneten Produkts bei einem Verhältnis zwischen 1,1 und 5.

**15.** Verfahren zur Herstellung der galenischen Form gemäß Anspruch 12,
dadurch **gekennzeichnet,**
daß es die folgenden Stufen umfaßt:
1) Erhalten eines Ansatzes des Wirkstoffs in einem flüssigen Medium, wobei der Wirkstoff in dem flüssigen Medium entweder löslich oder unlöslich sein kann und dessen Konzentration in dem flüssigen Medium geringer als oder gleich 10% (G/V) ist,
2) Versprühen des erhaltenen Ansatzes zur Gewinnung eines Aerosols im Strom des gasförmigen Fluidträgers,
3) Verdunsten des flüssigen Mediums des Aerosols, derart, daß die beim Trocknen des Aerosols resultierenden Mikroteilchen vom gasförmigen Fluidträger transportiert werden,
4) Filtrieren des gasförmigen Fluidträgers mit dem Gehalt an dem Wirkstoff in der Form von Mikroteilchen mittels eines Filters, das als Filterelement ein festes und poröses Material aufweist, das in trockenem Zustand erhalten worden ist durch Gefrieren, Zerkleinern, Gefriertrocknen und Verdichten, wodurch dem Material eine hinreichende Festigkeit zur Vermeidung eines Spaltens während des Durchtritts des gasförmigen Fluids durch seine Dickenerstreckung gegeben wurde, damit auf dem Filterelement die Mikroteilchen aufgefangen werden können.

**16.** Verfahren gemäß Anspruch 15, wobei der Wirkstoff in Wasser unlöslich, aber in einem organischen Lösungsmittel löslich ist,
dadurch **gekennzeichnet,**
daß es die folgenden Stufen umfaßt:

1) Erhalten einer Lösung des wasserunlöslichen Wirkstoffs in einem organischen Lösungsmittel, derart, daß die Konzentration an dem Wirkstoff geringer als oder gleich 10% (G/V) ist,

2) Versprühen der erhaltenen Lösung zu einem Aerosol in dem Strom des gasförmigen Fluidträgers,

3) Verdunsten des Lösungsmittels vom Wirkstoff im Strom des gasförmigen Fluidträgers, derart, daß die nach dem Trocknen des Aerosols erhaltenen festen Mikroteilchen vom gasförmigen Fluidträger tansportiert werden können,

4) Filtrieren des gasförmigen Fluidträgers mit dem Gehalt an dem Wirkstoff in der Form von im wesentlichen Mikroteilchen mit einer mittleren Kornverteilung zwischen 5 $\mu$m und 0,01 $\mu$m einerseits und dem Lösungsmittel für den Wirkstoff in der Form von Dampf andererseits mittels eines Filters, das als Filterelement ein festes, poröses und wasserlösliches Material enthält, das in trochenem Zustand erhalten worden ist durch Gefrieren, Zerkleinern, Gefriertrocknen und Verdichten zur Erzielung einer für die Vermeidung eines Spaltens während des Druchtritts des gasförmigen Fluids durch die Dickenerstreckung hinreichenden Festigkeit.

**17.** Verfahren gemäß Anspruch 15, wobei der Wirkstoff in Wasser löslich ist,
dadurch **gekennzeichnet,**
daß es die folgenden Stufen umfaßt:

1) Erhalten einer Lösung des Wirkstoffs in Wasser, derart, daß die Konzentration am Wirkstoff in der Lösung geringer als oder gleich 10% (G/V) ist,

2) Versprühen der so erhaltenen Lösung zur Gewinnung eines Aerosols im Strom des gasförmigen Fluidträgers,

3) Verdunsten des als Lösungsmittel für den Wirkstoff dienenden Wassers im Strom des gasförmigen Fluidträgers, derart, daß die nach dem Trocknen vom Aerosol resultierenden festen Mikroteilchen vom gasförmigen Fluidträger transportiert werden können,

4) Filtrieren des gasförmigen Fluidträgers mit dem Gehalt an den Mikroteilchen mit einer mittleren Korngrößenverteilung zwischen 5 $\mu$m und 0,01 $\mu$m einerseits und dem Lösungsmittel für den Wirkstoff in Form von Dampf andererseits mittels eines Filters, das als Filterelement ein festes und poröses Material enthält, das in trockenem Zustand erhalten worden ist durch Gefrieren, Zerkleinern, Gefriertrocknen und Verdichten zur Erzielung eines Materials mit zur Vermeidung eines Spaltens bei dem Durchtritt des gasförmigen Fluids durch die Dickenerstreckung hinreichenden Festigkeit.

**18.** Verfahren gemäß Anspruch 15, wobei der Wirkstoff in der Form von umhüllten Mikroteilchen vorliegt,
dadurch **gekennzeichnet,**
daß es die folgenden Stufen umfaßt:

1) Erhalten einer Suspension von Mikroteilchen des Wirkstoffs in einem flüssigen Medium mit einem Gehalt an einem gelösten Umhüllungsmaterial, wobei der Wirkstoff in dem flüssigen Medium unlöslich ist und die Konzentration des Wirkstoffs im flüssigen Medium geringer als oder gleich 10% (G/V) ist,

2) Versprühen der erhaltenen Suspension zur Herstellung eines eingehüllten Aerosols im Strom vom gasförmigen Fluidträger,

3) Verdunsten des flüssigen Mediums des Aerosols, derart, daß die beim Trocknen des Aerosols erhaltenen umhüllten Mikroteilchen vom gasförmigen Fluidträger transportiert werden,

4) Filtrieren des gasförmigen Fluidträgers mit dem Gehalt an den vom Umhüllungsmittel umhüllten Mikroteilchen mittels eines Filters, das als Filterelemnt ein festes und poröses Material enthält, das in trockenem Zustand erhalten worden ist durch Gefrieren, Zerkleinern, Gefriertrocknen und Verdichten zur Erzielung eines Materials mit zur Vermeidung eines Spaltens bei dem Durchtritt des gasförmigen Fluids durch die Dickenerstreckung hinreichenden Festigkeit, zwecks Auffangens der umhüllten Mikroteilchen auf dem Filterelement.

**19.** Verfahren gemäß irgendeinem der Ansprüche 16, 17 und 18,
dadurch **gekennzeichnet,**
daß man in der Stufe 1) einen flüssigen Ansatz herstellt, der einen Gehalt an dem Wirkstoff von weniger als oder gleich 5% (G/V) aufweist.

**20.** Verfahren gemäß irgendeinem der Ansprüche 16, 17 und 18,
dadurch **gekennzeichnet,**
daß das Versprühen gemäß der Stufe 2) derart durchgeführt wird, daß man Aerosolteilchen mit einem solchen mittleren Durchmesser erhält, daß nach dem Verdunsten des Lösungsmittels des flüssigen

27

Ansatzes feste Mikroteilchen mit einer mittleren Kornverteilchen zwischen 1 $\mu$m und 0,01 $\mu$m resultieren.

**21.** Verfahren gemäß irgendeinem der Ansprüche 16, 17 und 18,
dadurch **gekennzeichnet,**
daß das Verdunsten gemäß Stufe 3) bei einer Temperatur zwischen 25°C und 90°C durchgeführt wird.

**22.** Verfahren gemäß irgendeinem der Ansprüche 16, 17 und 18,
dadurch **gekennzeichnet,**
daß außerdem noch eine Stufe der Vermischung von dem das Filterelement und die Mirkroteilchen vom Wirkstoff umfassenden bei dem Filtrieren gemäß der Stufe 4) resultierenden Masse vorgesehen ist.

**23.** Verfahren gemäß Anspruch 18 mit der Gewinnung einer galenischen Form mit umhüllten Wirkstoffen,
dadurch **gekennzeichnet,**
daß außerdem noch vor der Stufe 1) die folgenden Stufen vorgesehen sind:
(a) Erhalten einer Lösung des Wirkstoffs in einem ersten Lösungsmittel, derart, daß die Konzentration desselben in dem ersten Lösungsmittel weniger als oder gleich 10% (G/V) ist,
(b) Versprühen der so erhaltenen Lösung in der Form eines flüssigen, vom Strom des gasförmigen Fluidträger transportierten Aerosols,
(c) Verdunsten des Lösungsmittels für den Wirkstoff im Strom des gasförmigen Fluidträgers, derart, daß nach der Trocknung der flüssigen Aerosolteilchen feste Mikroteilchen resultieren, die von dem gasförmigen Fluidträger fortbewegt werden,
(d) Filtrieren des gasförmigen Fluidträgers mit dem Gehalt an dem Wirkstoff in der Form von Mikroteilchen mit einer mittleren Kornverteilung zwischen 5 $\mu$m und 0,01 $\mu$m einerseits und dem Lösungsmittel für den Wirkstoff in der Form von Dampf andererseits mittels eines Filters, das als Filterelement ein festes und poröses Material enthält, das in dem trockenen Zustand erhalten worden ist durch Gefrieren, Zerkleinern, Gefriertrocknen und Verdichten zur Erzielung einer für das Vermeiden eines Spaltens bei dem Durchtritt des gasförmigen Fluidträgers durch die Dickenerstreckung hinreichenden mechanischen Festigkeit,
(e) Auflösen des für die Stufe (d) verwendeten Filterelements in einem zweiten Lösungsmittel entsprechend dem in Stufe 1) beschriebenen Vorgehen.

**24.** Anwendung des Verfahrens gemäß irgendeinem der Ansprüche 16, 17 und 18,
dadurch **gekennzeichnet,**
daß man die Mikroteilchen des Wirkstoffs im gegebenen Falle umhüllt, sammelt durch Auflösen der Matrix, die ihm zugeordnet ist und während des Filtrierens gemäß Stufe 4) als Filterelement diente, in einem geeigneten Lösungsmittel, das die gegebenenfalls umhüllten Mikroteilchen nicht auflöst.

**Patentansprüche für folgende Vertragsstaaten : ES, GR**

**1.** Filterelement für ein gasförmiges Fluid,
dadurch **gekennzeichnet,**
daß es in der Form einer porösen Schicht vorliegt und gebildet ist aus einem festen Material, das in dem trockenen Zustand erhalten wurde, durch (i) Gefrieren, (ii) Gefriertrocknung und dann (iii) Verdichtung zwecks Verleihung einer hinreichenden mechanischen Festigkeit zur weitgehenden Vermeidung eines Spaltens während des Durchtritts des gasförmigen Fluids durch die Dickenerstreckung hindurch.

**2.** Filterelement gemäß Anspruch 1,
dadurch **gekennzeichnet,**
daß es gebildet ist aus einem festen Material, das nach der Gefriertrocknung entsprechend einem Kompressionsverhältnis zwischen 1,1 und 5 verdichtet wurde.

**3.** Filterelement gemäß Anspruch 1,
dadurch **gekennzeichnet,**
daß es gebildet ist aus einem festen Material, das nach der Gefriertrocknung entsprechend einem Kompressionsverhältnis zwischen 1,2 und 4 verdichtet wurde.

**4.** Filterelement gemäß Anspruch 1,
dadurch **gekennzeichnet,**
daß es gebildet ist aus einem festen Material, das nach der Gefriertrocknung entsprechend einem Kompressionsverhältnis zwischen 1,25 und 3,60 verdichtet wurde.

**5.** Filterelement gemäß Anspruch 1,
dadurch **gekennzeichnet,**
daß es in seiner Masse ein Kohäsionsmittel enthält, das ausgewählt ist aus der Gruppe der plastifizierenden Substanzen.

**6.** Filterelement gemäß Anspruch 5,
dadurch **gekennzeichnet,**
daß das plastifizierende Kohäsionsmittel in der Masse des festen Materials vom Filterelement in einer Konzentration zwischen 1 und 75 Gew.-%, bezogen auf das Gesamtgewicht des festen Materials, vorliegt.

**7.** Filterelement gemäß irgendeinem der Ansprüche 1 bis 6,
dadurch **gekennzeichnet,**
daß es aus einem festen Material gebildet ist, das löslich oder dispergierbar in einem Lösungsmittel aus der Gruppe Wasser, nichtwässerige mineralische Lösungsmittel, organische Lösungsmittel und deren Mischungen ist.

**8.** Filterelement gemäß irgendeinem der Ansprüche 1 bis 7,
dadurch **gekennzeichnet,**
daß es gebildet ist aus einem festen Material, das in dem trockenen Zustand erhalten wird durch (a) Gefrieren, (b) Zerkleinern, (c) Gefriertrocknung und dann (d) Verdichtung.

**9.** Filter für ein gasförmiges Fluid, enthaltend ein Filterelement gemäß einem der Ansprüche 1 bis 8.

**10.** Verfahren zur Herstellung des Filterelements gemäß Anspruch 8,
dadurch **gekennzeichnet,**
daß man einen in flüssiger oder pastöser Form vorliegenden Ansatz aus einer polymeren Substanz und einem lösenden, verdünnenden oder dispergierenden Medium einer Temperatur von -40°C bis -80°C unterwirft, das erhaltene verfestigte und gefrorene Produkt zerkleinert, dann eine Gefriertrocknung des Mahlguts zum Sublimieren der Verteilungsflüssigkeit durchführt und das erhaltene gefriergetrocknete Produkt bei einem Verdichtungsverhältnis zwischen 1,1 und 5 verdichtet.

**11.** Verwendung eines Filterelements gemäß irgendeinem der Ansprüche 1 bis 8 auf dem Gebiet der Reinigung und Analyse von in einem gasförmigen Fluid enthaltenen Beimengungen,
dadurch **gekennzeichnet,**
daß man das gasförmige Fluid durch das in einem Filter angeordnete Filterelement bei einem Spannungsverlust zwischen 80 bis 10 000 Pa zum Durchströmen bringt.

**12.** Verfahren zur Herstellung einer galenischen Form, die in Assoziation enthält (i) ein Filterelement gemäß irgendeinem der Ansprüche 1 bis 8 als physiologisch akzeptable Matrix und wenigstens einen teilchenförmigen Wirkstoff, ausgewählt aus therapeutischen und kosmetischen Bestandteilen, wobei der als physiologisch akzeptable, an die Matrix gebundene Wirkstoff im wesentlichen von Mikroteilchen mit einer mittleren Kornverteilung zwischen 5 $\mu$m und 0,01 $\mu$m gebildet wird und ausgewählt ist aus Substanzen, die (1) löslich in Wasser, (2) unlöslich in Wasser oder (3) umhüllt sind,
dadurch **gekennzeichnet,**
daß es die folgenden Stufen umfaßt:
1) Erhalten eines Ansatzes des Wirkstoffs in einem flüssigen Medium, wobei der Wirkstoff in dem flüssigen Medium entweder löslich oder unlöslich sein kann und dessen Konzentration in dem flüssigen Medium geringer als oder gleich 10% (G/V) ist,
2) Versprühen des erhaltenen Ansatzes zur Gewinnung eines Aerosols im Strom des gasförmigen Fluidträgers,
3) Verdunsten des flüssigen Mediums des Aerosols, derart, daß die beim Trocknen des Aerosols resultierenden Mikroteilchen vom gasförmigen Fluidträger transportiert werden,

4) Filtrieren des gasförmigen Fluidträgers mit dem Gehalt an dem Wirkstoff in der Form von Mikroteilchen mittels eines Filters, das als Filterelement ein festes und poröses Material aufweist, das in trockenem Zustand erhalten worden ist durch Gefrieren, Zerkleinern, Gefriertrocknen und Verdichten, wodurch dem Material eine hinreichende Festigkeit zur Vermeidung eines Spaltens während des Durchtritts des gasförmigen Fluids durch seine Dickenerstreckung gegeben wurde, damit auf dem Filterelement die Mikroteilchen aufgefangen werden können.

13. Verfahren gemäß Anspruch 12, wobei der Wirkstoff in Wasser unlöslich, aber in einem organischen Lösungsmittel löslich ist,

dadurch **gekennzeichnet,**

daß es die folgenden Stufen umfaßt:

1) Erhalten einer Lösung des wasserunlöslichen Wirkstoffs in einem organischen Lösungsmittel, derart, daß die Konzentration an dem Wirkstoff geringer als oder gleich 10% (G/V) ist,

2) Versprühen der erhaltenen Lösung zu einem Aerosol in dem Strom des gasförmigen Fluidträgers,

3) Verdunsten des Lösungsmittels vom Wirkstoff im Strom des gasförmigen Fluidträgers, derart, daß die nach dem Trocknen des Aerosols erhaltenen festen Mikroteilchen vom gasförmigen Fluidträger transportiert werden können,

4) Filtrieren des gasförmigen Fluidträgers mit dem Gehalt an dem Wirkstoff in der Form von im wesentlichen Mikroteilchen mit einer mittleren Kornverteilung zwischen 5 $\mu$m und 0,01 $\mu$m einerseits und dem Lösungsmittel für den Wirkstoff in der Form von Dampf andererseits mittels eines Filters, das als Filterelement ein festes, poröses und wasserlösliches Material enthält, das in trochenem Zustand erhalten worden ist durch Gefrieren, Zerkleinern, Gefriertrocknen und Verdichten zur Erzielung einer für die Vermeidung eines Spaltens während des Druchtritts des gasförmigen Fluids durch die Dickenerstreckung hinreichenden Festigkeit.

14. Verfahren gemäß Anspruch 12, wobei der Wirkstoff in Wasser löslich ist,

dadurch **gekennzeichnet,**

daß es die folgenden Stufen umfaßt:

1) Erhalten einer Lösung des Wirkstoffs in Wasser, derart, daß die Konzentration am Wirkstoff in der Lösung geringer als oder gleich 10% (G/V) ist,

2) Versprühen der so erhaltenen Lösung zur Gewinnung eines Aerosols im Strom des gasförmigen Fluidträgers,

3) Verdunsten des als Lösungsmittel für den Wirkstoff dienenden Wassers im Strom des gasförmigen Fluidträgers, derart, daß die nach dem Trocknen vom Aerosol resultierenden festen Mikroteilchen vom gasförmigen Fluidträger transportiert werden können,

4) Filtrieren des gasförmigen Fluidträgers mit dem Gehalt an den Mikroteilchen mit einer mittleren Korngrößenverteilung zwischen 5 $\mu$m und 0,01 $\mu$m einerseits und dem Lösungsmittel für den Wirkstoff in Form von Dampf andererseits mittels eines Filters, das als Filterelement ein festes und poröses Material enthält, das in trockenem Zustand erhalten worden ist durch Gefrieren, Zerkleinern, Gefriertrocknen und Verdichten zur Erzielung eines Materials mit zur Vermeidung eines Spaltens bei dem Durchtritt des gasförmigen Fluids durch die Dickenerstreckung hinreichenden Festigkeit.

15. Verfahren gemäß Anspruch 12, wobei der Wirkstoff in der Form von umhüllten Mikroteilchen vorliegt,

dadurch **gekennzeichnet,**

daß es die folgenden Stufen umfaßt:

1) Erhalten einer Suspension von Mikroteilchen des Wirkstoffs in einem flüssigen Medium mit einem Gehalt an einem gelösten Umhüllungsmaterial, wobei der Wirkstoff in dem flüssigen Medium unlöslich ist und die Konzentration des Wirkstoffs im flüssigen Medium geringer als oder gleich 10% (G/V) ist,

2) Versprühen der erhaltenen Suspension zur Herstellung eines eingehüllten Aerosols im Strom vom gasförmigen Fluidträger,

3) Verdunsten des flüssigen Mediums des Aerosols, derart, daß die beim Trocknen des Aerosols erhaltenen umhüllten Mikroteilchen vom gasförmigen Fluidträger transportiert werden,

4) Filtrieren des gasförmigen Fluidträgers mit dem Gehalt an den vom Umhüllungsmittel umhüllten Mikroteilchen mittels eines Filters, das als Filterelemnt ein festes und poröses Material enthält, das in trockenem Zustand erhalten worden ist durch Gefrieren, Zerkleinern, Gefriertrocknen und Verdichten zur Erzielung eines Materials mit zur Vermeidung eines Spaltens bei dem Durchtritt des gasförmigen Fluids durch die Dickenerstreckung hinreichenden Festigkeit, zwecks Auffangens der umhüllten Mikroteilchen auf dem Filterelement.

**16.** Verfahren gemäß irgendeinem der Ansprüche 13, 14 und 15,
dadurch **gekennzeichnet,**
daß man in der Stufe 1) einen flüssigen Ansatz herstellt, der einen Gehalt an dem Wirkstoff von weniger als oder gleich 5% (G/V) aufweist.

**17.** Verfahren gemäß irgendeinem der Ansprüche 13, 14 und 15,
dadurch **gekennzeichnet,**
daß das Versprühen gemäß der Stufe 2) derart durchgeführt wird, daß man Aerosolteilchen mit einem solchen mittleren Durchmesser erhält, daß nach dem Verdunsten des Lösungsmittels des flüssigen Ansatzes feste Mikroteilchen mit einer mittleren Kornverteilchen zwischen 1 $\mu$m und 0,01 $\mu$m resultieren.

**18.** Verfahren gemäß irgendeinem der Ansprüche 13, 14 und 15,
dadurch **gekennzeichnet,**
daß das Verdunsten gemäß Stufe 3) bei einer Temperatur zwischen 25°C und 90°C durchgeführt wird.

**19.** Verfahren gemäß irgendeinem der Ansprüche 13, 14 und 15,
dadurch **gekennzeichnet,**
daß außerdem noch eine Stufe der Vermischung von dem das Filterelement und die Mirkroteilchen vom Wirkstoff umfassenden bei dem Filtrieren gemäß der Stufe 4) resultierenden Masse vorgesehen ist.

**20.** Verfahren gemäß Anspruch 15 mit der Gewinnung einer galenischen Form mit umhüllten Wirkstoffen,
dadurch **gekennzeichnet,**
daß außerdem noch vor der Stufe 1) die folgenden Stufen vorgesehen sind:
(a) Erhalten einer Lösung des Wirkstoffs in einem ersten Lösungsmittel, derart, daß die Konzentration desselben in dem ersten Lösungsmittel weniger als oder gleich 10% (G/V) ist,
(b) Versprühen der so erhaltenen Lösung in der Form eines flüssigen, vom Strom des gasförmigen Fluidträger transportierten Aerosols,
(c) Verdunsten des Lösungsmittels für den Wirkstoff im Strom des gasförmigen Fluidträgers, derart, daß nach der Trocknung der flüssigen Aerosolteilchen feste Mikroteilchen resultieren, die von dem gasförmigen Fluidträger fortbewegt werden,
(d) Filtrieren des gasförmigen Fluidträgers mit dem Gehalt an dem Wirkstoff in der Form von Mikroteilchen mit einer mittleren Kornverteilung zwischen 5 $\mu$m und 0,01 $\mu$m einerseits und dem Lösungsmittel für den Wirkstoff in der Form von Dampf andererseits mittels eines Filters, das als Filterelement ein festes und poröses Material enthält, das in dem trockenen Zustand erhalten worden ist durch Gefrieren, Zerkleinern, Gefriertrocknen und Verdichten zur Erzielung einer für das Vermeiden eines Spaltens bei dem Durchtritt des gasförmigen Fluidträgers durch die Dickenerstrekkung hinreichenden mechanischen Festigkeit,
(e) Auflösen des für die Stufe (d) verwendeten Filterelements in einem zweiten Lösungsmittel entsprechend dem in Stufe 1) beschriebenen Vorgehen.

**21.** Anwendung des Verfahrens gemäß irgendeinem der Ansprüche 13, 14 und 15,
dadurch **gekennzeichnet,**
daß man die Mikroteilchen des Wirkstoffs im gegebenen Falle umhüllt, sammelt durch Auflösen der Matrix, die ihm zugeordnet ist und während des Filtrierens gemäß Stufe 4) als Filterelement diente, in einem geeigneten Lösungsmittel, das die gegebenenfalls umhüllten Mikroteilchen nicht auflöst.

FIG_1

FIG_2

FIG_3